# EUROPÄISCHE PATENTSCHRIFT

(11) **EP 1 922 299 B1**
(45) Veröffentlichungstag und Bekanntmachung des Hinweises auf die Patenterteilung: **24.11.2010**
(21) Anmeldenummer: 06792887.9
(22) Anmeldetag: 18.08.2006
(51) Int. Cl.: C07C 51/41, C07F 5/06, C01B 3/00

(54) **VERFAHREN ZUR HERSTELLUNG VON METALLORGANISCHEN GERÜSTMATERIALIEN HAUPTGRUPPEN METALLIONEN ENTHALTEND**
METHOD FOR PRODUCING ORGANOMETALLIC FRAMEWORK MATERIALS CONTAINING MAIN GROUP METAL IONS
PROCEDE POUR PRODUIRE DES MATERIAUX A STRUCTURE ORGANOMETALLIQUE COMPORTANT DES IONS METALLIQUES DU GROUPE PRINCIPAL

(30) Priorität: 22.08.2005 DE 102005039623
(43) Veröffentlichungstag der Anmeldung: 21.05.2008
(73) Patentinhaber: BASF SE, 67056 Ludwigshafen (DE)
(72) Erfinder: SCHUBERT, Markus, 67063 Ludwigshafen (DE); MÜLLER, Ulrich, 67435 Neustadt (DE); TONIGOLD, Markus, 89134 Blaustein (DE); RUETZ, Roger, 68163 Mannheim (DE)
(86) Internationale Anmeldenummer: PCT/EP2006/065442
(87) Internationale Veröffentlichungsnummer: WO 2007/023134

(56) Entgegenhaltungen:
- EP-A2- 0 790 253
- JP-A- 59 155 333

## Beschreibung

Die vorliegende Erfindung betrifft ein Verfahren zur Herstellung eines porösen metallorganischen Gerüstmaterials sowie die Verwendung der hergestellten Gerüstmaterialien.

Poröse metallorganische Gerüstmaterialien sind im Stand der Technik bekannt. Diese enthalten typischerweise mindestens eine an mindestens ein Metallion koordinativ gebundene mindestens zweizähnige organische Verbindung. Solche metallorganischen Gerüstmaterialien (MOF = metal organic framework) werden beispielsweise in US-A 5,648,508, EP-A 0 790 253, M.O. Keeffe, J. Sol. State Chem., 152 (2000), 3 - 20; H. Li et al., Nature 402 (1999), 276; M. Eddaoudi, Topics in catalysis 9 (1999), 105-111; B. Chen et al., Science 291 (2001), 1021-1023 und DE-A 101 11 230 beschrieben.

Für die Herstellung solcher porösen metallorganischen Gerüstmaterialien sind zahlreiche Verfahren entwickelt worden. Typischerweise wird hierbei in einem geeigneten Lösemittel ein Metallsalz mit der mindestens zweizähnigen organischen Verbindung, wie beispielsweise einer Dicarbonsäure, unter erhöhtem Druck und erhöhter Temperatur umgesetzt.

Dabei treten jedoch häufig Schwierigkeiten auf. Ein Problem kann darin bestehen, dass aufgrund der Verwendung eines Metallsalzes, das nach Bildung des metallorganischen Gerüstmaterials im Reaktionsmedium verbleibende Gegenion zum Metallkation (zum Beispiel Nitrat) vom Gerüstmaterial abgetrennt werden muss.

Durch die Verwendung hoher Drücke und Temperaturen wird an die Syntheseapparatur zur Herstellung eines porösen metallorganischen Gerüstmaterials hohe Anforderungen gestellt. Es ist üblicherweise nur eine Batch-Synthese in vergleichsweise kleinen Apparaturen möglich und beschrieben. Ein Scale-up erweist sich als sehr aufwendig.

Eine weitere Schwierigkeit besteht darin, dass je nach verwendetem Metall und organischer Verbindung zur Herstellung des Gerüstmaterials eine Übertragbarkeit der Reaktionsbedingungen nicht ohne weiteres möglich ist. Solch ein Fall liegt beispielsweise dann vor, wenn die Metallkomponente im metallorganischen Gerüstmaterial ein Hauptgruppenmetall der zweiten oder dritten Hauptgruppe des Periodensystems ist. Hierbei werden zur Herstellung teilweise deutlich unterschiedliche Reaktionsbedingungen im Vergleich zu analogen Gerüstmaterialien, bei denen die Metallkomponente ein Übergangsmetall, beispielsweise Zink oder Kupfer, ist, angewendet.

Solche porösen metallorganischen Gerüstmaterialien, die ein Hauptgruppenmetall der zweiten oder dritten Hauptgruppe aufweisen können, unterscheiden sich auch im Hinblick auf ihre Eigenschaften von den oben genannten analogen Gerüstmaterialien, was ein Grund dafür sein könnte, dass hier im Stand der Technik häufig modifizierte Herstellverfahren herangezogen werden.

T. Loiseau et al., Chem. Eur. J. 2004, (10), 1373-1382 beschreibt beispielsweise die Herstellung porösen Aluminiumterephthalats. Dieses zeichnet sich durch eine bemerkenswert hohe thermische Stabilität aus. So wird berichtet, dass das Material sich erst bei einer Temperatur ab 500 °C zersetzt. Die Synthese erfolgt hierbei durch Umsetzung von Aluminiumnitrat (als Nonahydrat) und 1,4-Benzoldicarbonsäure in deionisiertem Wasser. Die Reaktion erfolgt unter Druck bei 220 °C. Im Gegensatz zu anderen metallorganischen Gerüstmaterialien zeigt sich hier bei der Herstellung, dass der verwendete Ligand als organische Verbindung nicht nur im metallorganischen Gerüstmaterial als Gerüstbaustein auftritt, sondern zudem als Ligand in den Poren des Gerüstmaterials gehalten wird. Diese zusätzliche Bindung des Liganden im porösen metallorganischen Gerüstmaterial tritt typischerweise bei analogen Gerüstmaterialien, die aus Übergangsmetallen aufgebaut sind, nicht auf. Zur Lösung dieses Problems schlagen Loiseau et al. vor, das Material so lange zu erhitzen, bis der in den Poren festgehaltene Ligand entfernt ist. Diese Vorgehensweise birgt den Nachteil in sich, dass der eigentlichen Synthese ein teilweise zeitraubender und stringenter Nachbehandlungsschritt folgen soll, der stark von dem Siede- bzw. Dampfdruck des Liganden abhängig ist. Bei besonders hoch siedenden Liganden kann diese Vorgehensweise auch gänzlich fehlschlagen.

Z. Lin et al., Inorganic Chemistry 44 (2005), 73-76 beschreiben ein metallorganisches Gerüstmaterial, wobei Indium die Metallkomponente bildet und als organische Verbindung 1,3,5-Benzoltricarbonsäure verwendet wird. Hier wird ebenfalls Wasser als Lösemittel verwendet. Weiterhin weisen Lin et al. darauf hin, dass zur Reaktion ein hoher Überschuss einer Base (Pyridin) zugesetzt sein muss, um einen adäquaten pH-Wert einzustellen.

L. Pan et al., Inorg. Chem. 40 (2001), 1271-1283, beschreiben Calcium, Strontium und Barium enthaltende poröse metallorganische Gerüstmaterialien und schlagen ebenfalls die Verwendung von Wasser beziehungsweise Wasser/Triethylamin vor. Als Ligand wird 3,5-Pyrazoldicarbonsäure verwendet.

Z. Fei et al., Inorg. Chem. 44 (2005), 5200-5202, beschreiben Strontium-Imidazolium Carboxylate.

H.-F. Zhu et al., Crystal Growth & Design 5 (2005), 177-182 beschreiben metallorganische Gerüstmaterialien aus Calcium und Barium mit 1,3,5-Benzoltricarbonsäure in Wasser. J. Sun et al., Angew. Chem. 114 (2002), 4651-4653 beschreiben unter anderem die Herstellung von Indiumterephthalat in DMF. Die Reaktion findet hierbei unter erhöhtem Druck bei 160 °C statt.

B. Gomez-Lor et al., Inorganic Chemistry 41 (2002), 2429-2432, beschreiben ebenfalls Indiumterephthalat, wobei die Reaktion in Wasser unter Verwendung von Triethylamin als Base durchgeführt wird.

Nachteilig bei der Verwendung von Wasser ist die teilweise schlechte Löslichkeit der organischen Verbindung in Wasser sowie die häufig erhaltenen geringen Ausbeuten an Gerüstmaterial und/oder dessen geringe spezifische Oberfläche.

Ein weiterer Nachteil liegt in der Verwendung hoher Drücke. Hier erlaubt der apparativ hohe Aufwand meist kein Rühren zur Durchmischung der einzelnen Ausgangsstoffe zur Herstellung des metallorganischen Gerüstmaterials.

Es besteht somit nach wie vor ein Bedarf für verbesserte Verfahren zur Herstellung von porösen metallorganischen Gerüstmaterialien, bei denen die Metallkomponente ein Metall der zweiten oder dritten Hauptgruppe des Periodensystems ist.

Die Aufgabe der vorliegenden Erfindung liegt somit darin, ein verbessertes Verfahren zur Herstellung solcher porösen metallorganischen Gerüstmaterialien bereitzustellen.

Die Aufgabe wird gelöst durch ein Verfahren zur Herstellung eines porösen metallorganischen Gerüstmaterials den Schritt enthaltend
Umsetzung mindestens einer Metallverbindung mit mindestens einer mindestens zweizähnigen organischen Verbindung, die koordinativ an das Metall binden kann, in Gegenwart eines nicht-wässrigen organischen Lösemittels, wobei das Metall Be^{II}, Mg^{II}, Ca^{II}, Sr^{II}, Ba^{II}, Al^{III}, Ga^{III} oder In^{III} ist und wobei die organische Verbindung zumindest zwei Atome jeweils unabhängig ausgewählt aus der Gruppe bestehend aus Sauerstoff, Schwefel und Stickstoff aufweist, über die die organische Verbindung an das Metall koordinativ binden kann, wobei die Umsetzung unter Rühren und bei einem Druck von höchstens 2 bar (absolut) erfolgt und wobei nach der Umsetzung ein Calcinierungsschritt bei mehr als 250°C erfolgt.

Es wurde nämlich gefunden, dass durch die Verwendung eines nicht-wässrigen organischen Lösemittels und den weiter genannten Bedingungen eine zumindest teilweise effizientere Herstellung eines porösen metallorganischen Gerüstmaterials möglich ist, die insbesondere ein einfaches "Scale up" ermöglicht.

Hierbei ist unter anderem vorteilhaft, dass die Umsetzung unter Rühren stattfinden kann, was auch bei einem Scale-up vorteilhaft ist.

Die Umsetzung erfolgt bei einem Druck von höchstens 2 bar (absolut). Vorzugsweise beträgt der Druck jedoch höchstens 1230 mbar (absolut). Insbesondere bevorzugt findet die Umsetzung bei Atmosphärendruck statt.

Die Umsetzung kann bei Raumtemperatur durchgeführt werden. Vorzugsweise findet diese jedoch bei Temperaturen oberhalb der Raumtemperatur statt. Vorzugsweise beträgt die Temperatur mehr als 100°C. Weiterhin bevorzugt beträgt die Temperatur höchstens 180°C und mehr bevorzugt höchstens 150°C.

Typischerweise werden die oben beschriebenen metallorganischen Gerüstmaterialien in Wasser als Lösemittel unter Zusatz einer weiteren Base durchgeführt. Dies dient insbesondere dazu, dass bei Einsatz einer mehrbasigen Carbonsäure als mindestens zweizähniger organischer Verbindung diese leicht löslich in Wasser ist. Durch die Verwendung des nicht-wässrigen organischen Lösemittels ist es nicht erforderlich, eine solche Base einzusetzen. Nichts desto trotz kann das Lösemittel für das erfindungsgemäße Verfahren derart gewählt werden, dass dieses als solches basisch reagiert, was jedoch nicht zwingend für die Durchführung des erfindungsgemäßen Verfahrens sein muss.

Ebenso kann eine Base eingesetzt werden. Bevorzugt ist jedoch, dass keine zusätzliche Base eingesetzt wird.

Weiterhin bevorzugt kann die verwendete Metallverbindung zur Herstellung des porösen metallorganischen Gerüstmaterials nicht-ionisch sein und/oder das Gegenion zum Metallkation kann sich von einem protischen Lösemittel ableiten lassen. Durch die Verwendung einer nicht-ionischen Verbindung kann bei geeigneter Wahl vermieden werden, dass bei der Umsetzung zum porösen metallorganischen Gerüstmaterial das Metall in Form eines Salzes vorliegt und dadurch gegebenenfalls Schwierigkeiten bei der Entfernung des korrespondierenden Anions im Metallsalz auftreten, sofern durch die Metallverbindung bei der Umsetzung keine weiteren störenden Salze erzeugt werden. Stellt das Gegenion ein Lösemittelanion dar, kann dieses bei geeigneter Wahl nach Umsetzung als Lösemittel vorliegen, das gleich dem eingesetzten nicht-wässrigen organischen Lösemittel oder verschieden davon sein kann. In letzterem Fall ist bevorzugt, wenn dieses Lösemittel mit dem nicht-wässrigen organischen Lösemittel zumindest zum Teil mischbar ist. Sollte bei der Umsetzung der Metallverbindung Wasser entstehen, sollte dessen Anteil in den weiter unten beschriebenen Grenzen liegen. Dies kann dadurch erreicht werden, dass eine ausreichende Menge des nicht-wässrigen organischen Lösemittels eingesetzt wird.

Solche nicht-ionischen Verbindungen bzw. Gegenionen zum Metallkation, die sich von protischen Lösemitteln ableiten lassen, können beispielsweise Metallalkoholate, beispielsweise Methanolate, Ethanolate, Propanolate, Butanolate, insbesondere Aluminiumalkoholate sein. Ebenso sind Oxide oder Hydroxide denkbar.

Bei dem eingesetzten Metall handelt es sich um Be^{II}, Mg^{II}, Ca^{II}, Sr^{II}, Ba^{II}, Al^{III}, Ga^{III} oder In^{III}. Vorzugsweise handelt es sich bei dem Metall um Mg^{II}, Ca^{II}, Sr^{II}, Ba^{II}, Al^{III} oder In^{III}. Ganz besonders bevorzugt sind Al^{III} und Mg^{II}. Insbesondere bevorzugt ist Al^{III}.

Die mindestens eine mindestens zweizähnige organische Verbindung weist zumindest zwei Atome auf, die jeweils unabhängig ausgewählt sind aus der Gruppe bestehend aus Sauerstoff, Schwefel und Stickstoff, über die die organische Verbindung an das Metall koordinativ binden kann. Diese Atome können Teil des Gerüstes oder funktioneller Gruppen sein.

Als funktionelle Gruppen, über die die genannten koordinativen Bindungen ausgebildet werden können, sind insbesondere beispielsweise folgende funktionellen Gruppen zu nennen: -CO₂H, -CS₂H, -COSH, -NO₂, -SO₃H, -Ge(OH)₃, -Sn(OH)₃, -Si(SH)₄, -Ge(SH)₄, -Sn(SH)₃, -AsO₃H, -AsO₄H, -P(SH)₃, -As(SH)₃, -CH(RSH)₂, -C(RSH)₃, -CH(RNH₂)₂, -C(RNH₂)₃, -CH(ROH)₂, -C(ROH)₃, -CH(RCN)₂, -C(RCN)₃ wobei R beispielsweise bevorzugt eine Alkylengruppe mit 1, 2, 3, 4 oder 5 Kohlenstoffatomen wie beispielsweise eine Methylen-, Ethylen-, n-Propylen-, i-Propylen, n-Butylen-, i-Butylen-, tert-Butyten- oder n-Pentylengruppe, oder eine Arylgruppe, enthaltend 1 oder 2 aromatische Kerne wie beispielsweise 2 C₆-Ringe, die gegebenenfalls kondensiert sein können und unabhängig voneinander mit mindestes jeweils einem Substituenten geeignet substituiert sein können, und/oder die unabhängig voneinander jeweils mindestens ein Heteroatom wie beispielsweise N, O und/oder S enthalten können. Gemäß ebenfalls bevorzugten Ausführungsformen sind funktionelle Gruppen zu nennen, bei denen der oben genannte Rest R nicht vorhanden ist. Diesbezüglich sind unter anderem - CH(SH)₂, -C(SH)₃, -CH(NH₂)₂, -C(NH₂)₃, -CH(OH)₂, -C(OH)₃, -CH(CN)₂ oder -C(CN)₃ zu nennen.

Die mindestens zwei funktionellen Gruppen können grundsätzlich an jede geeignete organische Verbindung gebunden sein, solange gewährleistet ist, dass die diese funktionellen Gruppen aufweisende organische Verbindung zur Ausbildung der koordinativen Bindung und zur Herstellung des Gerüstmaterials befähigt ist.

Bevorzugt leiten sich die organischen Verbindungen, die die mindestens zwei funktionellen Gruppen enthalten, von einer gesättigten oder ungesättigten aliphatischen Verbindung oder einer aromatischen Verbindung oder einer sowohl aliphatischen als auch aromatischen Verbindung ab.

Die aliphatische Verbindung oder der aliphatische Teil der sowohl aliphatischen als auch aromatischen Verbindung kann linear und/oder verzweigt und/oder cyclisch sein, wobei auch mehrere Cyden pro Verbindung möglich sind. Weiter bevorzugt enthält die aliphatische Verbindung oder der aliphatische Teil der sowohl aliphatischen als auch aromatischen Verbindung 1 bis 15, weiter bevorzugt 1 bis 14, weiter bevorzugt 1 bis 13, weiter bevorzugt 1 bis 12, weiter bevorzugt 1 bis 11 und insbesondere bevorzugt 1 bis 10 C-Atome wie beispielsweise 1, 2, 3, 4, 5, 6, 7, 8, 9 oder 10 C-Atome. Insbesondere bevorzugt sind hierbei unter anderem Methan, Ethan, Butan, 2-Buten, Adamantan, Acetylen, Ethylen oder Butadien.

Die aromatische Verbindung oder der aromatische Teil der sowohl aromatischen als auch aliphatischen Verbindung kann einen oder auch mehrere Kerne wie beispielsweise zwei, drei, vier oder fünf Kerne aufweisen, wobei die Kerne getrennt voneinander und/oder mindestens zwei Kerne in kondensierter Form vorliegen können. Besonders bevorzugt weist die aromatische Verbindung oder der aromatische Teil der sowohl aliphatischen als auch aromatischen Verbindung einen, zwei oder drei Kerne auf, wobei einer oder zwei Kerne besonders bevorzugt sind. Unabhängig voneinander kann weiter jeder Kern der genannten Verbindung mindestens ein Heteroatom wie beispielsweise N, O, S, B, P, Si, bevorzugt N, O und/oder S enthalten. Weiter bevorzugt enthält die aromatische Verbindung oder der aromatische Teil der sowohl aromatischen als auch aliphatischen Verbindung einen oder zwei C₆-Kerne, wobei die zwei entweder getrennt voneinander oder in kondensierter Form vorliegen. Insbesondere sind als aromatische Verbindungen Benzol, Pyren, Dihydropyren, Naphthalin und/oder Biphenyl und/oder Bipyridyl und/oder Pyridyl zu nennen.

Beispielsweise sind unter anderem trans-Muconsäure oder Fumarsäure oder Phenylenbisacrylsäure zu nennen.

Beispielsweise sind im Rahmen der vorliegenden Erfindung Dicarbonsäuren wie etwa Oxalsäure, Bernsteinsäure, Weinsäure, 1,4-Butandicarbonsäure, 1,4-Butendicarbonsäure, 4-Oxo-Pyran-2,6-dicarbonsäure, 1,6-Hexandicarbonsäure, Decandicarbonsäure, 1,8-Heptadecandicarbonsäure, 1,9-Heptadecandicarbonsäure, Heptadecandicarbonsäure, Acetylendicarbonsäure, 1,2-Benzoldicarbonsäure, 1,3-Benzoldicarbonsäure, 2,3-Pyridindicarbonsäure, Pyridin-2,3-dicarbonsäure, 1,3-Butadien-1,4-dicarbonsäure, 1,4-Benzoldicarbonsäure, p-Benzoldicarbonsäure, Imidazol-2,4-dicarbonsäure, 2-Methyl-chinolin-3,4-dicarbonsäure, Chinolin-2,4-dicarbonsäure, Chinoxalin-2,3-dicarbonsäure, 6-Chlorchinoxalin-2,3-dicarbonsäure, 4,4'-Diaminphenylmethan-3,3'-dicarbonsäure, Chinolin-3,4-dicartionsäure, 7-Chlor-4-hydroxychinolin-2,8-dicarbonsäure, Diimiddicarbonsäure, Pyridin-2,6-dicarbonsäure, 2-Methylimidazol-4,5-dicarbonsäure, Thiophen-3,4-dicarbonsäure, 2-Isopropylimidazol-4,5-dicarbonsäure, Tetrahydropyran-4,4-dicarbonsäure, Perylen-3,9-dicarbonsäure, Perylendicarbonsäure, Pluriol E 200-dicarbonsäure, 3,6-Dioxaoctandicarbonsäure, 3,5-Cydohexadien-1,2-dicarbonsäure, Octadicarbonsäure, Pentan-3,3-carbonsäure, 4,4'-Diamino-1,1'-diphenyl-3,3'-dicarbon-säure, 4,4'-Diaminodiphenyl-3,3'-dicarbonsäure, Benzidin-3,3'-dicarbonsäure, 1,4-Bis-(phenylamino)-benzol-2,5-dicarbonsäure, 1,1'-Dinaphthyldicar bonsäure, 7-Chlor-8-methylchinolin-2,3-dicarbonsäure, 1-Anilinoanthrachinon-2,4'-dicarbonsäure, Polytetrahydrofuran-250-dicarbonsäure, 1,4-Bis-(carboxymethyl)-piperazin-2,3-dicarbonsäure, 7-Chlorchinolin-3,8-dicarbonsäure, 1-(4-Carboxy)phenyl-3-(4-chlor)-phenylpyrazolin-4,5-dicarbonsäure, 1,4,5,6,7,7,-Hexachlor-5-norbomen-2,3-dicarbonsäure, Phenylindandicarbonsäure, 1,3-Dibenzyl-2-oxo-imidazolidin-4,5-dicarbonsäure, 1,4-Cyclohexandicarbonsäure, Naphthalin-1,8-dicarbonsäure, 2-Benzoyl-benzol-1,3-dicarbon-säure, 1,3-Dibenzyl-2-oxoimidazolidin-4,5-cis-dicarbonsäure, 2,2'-Bichinolin-4,4¹-di-carbonsäure, Pyridin-3,4-dicarbonsäure, 3,6,9-Trioxaundecandicarbonsäure, Hydroxybenzophenon-dicarbonsäure, Pluriol E 300-dicarbonsäure, Pluriol E 400-dicarbonsäure, Pluriol E 600-dicarbonsäure, Pyrazol-3,4-dicarbonsäure, 2,3-Pyrazindicarbonsäure, 5,6-Dimethyl-2,3-pyrazindicarbonsäure, 4,4'-Diaminodiphenyletherdiimiddicarbonsäure, 4,4'-Diaminodiphenylmethandiimiddicarbonsäure, 4,4'-Diaminodiphenylsulfondiimiddicarbonsäure, 1,4-Naphthalindicarbonsäure, 2,6-Naphthalindicarbonsäure, 1,3-Adamantandicarbonsäure, 1,8-Naphthalindicarbonsäure, 2,3-Naphthalindicarbonsäure, 8-Methoxy-2,3-naphthalindicarbonsäure, 8-Nitro-2,3-naphthalincarbonsäure, 8-Sulfo-2,3-naphthalindicarbonsäure, Anthracen-2,3-dicarbonsäure, 2',3'-Diphenyl-p-terphenyl-4,4"-dicarbonsäure, Diphenylether-4,4'-dicarbonsäure, Imidazol-4,5-dicarbonsäure, 4(1H)-Oxothiochromen-2,8-dicarbonsäure, 5-tert-Butyl-1,3-benzoldicarbonsäure, 7,8-Chinolindicarbonsäure, 4,5-lmidazoldicarbonsäure, 4-Cyclohexen-1,2-dicarbonsäure, Hexatriacontandicarbonsäure, Tetradecandicarbonsäure, 1,7-Heptadicarbonsäure, 5-Hydroxy-1,3-Benzoldicarbonsäure, 2,5-Dihydroxy-1,4-dicarbonsäure, Pyrazin-2,3-dicarbonsäure, Furan-2,5-dicarbonsäure, 1-Nonen-6,9-dicarbonsäure, Eicosendicarbonsäure, 4,4'-Dihydroxydiphenylmethan-3,3'-dicarbonsäure, 1-Amino-4-methyl-9,10-dioxo-9,10-dihydroanthracen-2,3-dicarbonsäure, 2,5-Pyridindicarbonsäure, Cyclohexen-2,3-dicarbonsäure,2,9-Dichlorfluorubin-4,11-dicarbonsäure, 7-Chlor-3-methylchinolin-6,8-dicarbonsäure, 2,4-Dichlorbenzophenon-2',5'-dicarbonsäure, 1,3-Benzoldicarbonsäure, 2,6-Pyridindicarbonsäure, 1-Methylpyrrol-3,4-dicarbonsäure, 1-Benzyl-1H-pyrrol-3,4-dicarbonsäure, Anthrachinon-1,5-dicarbonsäure, 3,5-Pyrazoldicarbonsäure, 2-Nitrobenzol-1,4-dicarbonsäure, Heptan-1,7-dicarbonsäure, Cyclobutan-1,1-dicarbonsäure 1,14-Tetradecandicarbonsäure, 5,6-Dehydronorboman-2,3-dicarbonsäure, 5-Ethyl-2,3-pyridindicarbonsäure oder Campherdicarbonsäure,
Tricarbonsäuren wie etwa

2-Hydroxy-1,2,3-propantricarbonsäure, 7-Chlor-2,3,8-chinolintricarbonsäure, 1,2,3-, 1,2,4-Benzoltricarbonsäure, 1,2,4-Butantricarbonsäure, 2-Phosphono-1,2,4-butantricarbonsäure, 1,3,5-Benzoltricarbonsäure, 1-Hydroxy-1,2,3-Propantricarbonsäure, 4,5-Dihydro-4,5-dioxo-1H-pyrrolo[2,3-F]chinolin-2,7,9-tricarbonsäure, 5-Acetyl-3-amino-6-methylbenzol-1,2,4-tricarbonsäure, 3-Amino-5-benzoyl-6-methylbenzol-1,2,4-tricarbon-+säure, 1,2,3-Propantricarbonsäure oder Aurintricarbonsäure,
oder Tetracarbonsäuren wie etwa

1,1-Dioxidperylo[1,12-BCD]thiophen-3,4,9,10-tetracarbonsäure, Perylentetracarbonsäuren wie Perylen-3,4,9,10-tetracarbonsäure oder oder Perylen-1,12-sulfon-3,4,9,10-tetracarbonsäure, Butantetracarbonsäuren wie 1,2,3,4-Butantetracarbonsäure oder Meso-1,2,3,4-Butantetracarbonsäure, Decan-2,4,6,8-tetracarbonsäure, 1,4,7,10,13,16-Hexaoxacyclooctadecan-2,3,11,12-tetracarbonsäure, 1,2,4,5-Benzoltetracarbonsäure, 1,2,11,12-Dodecantetracarbonsäure, 1,2,5,6-Hexan-tetracarbonsäure, 1,2,7,8-Octantetracarbonsäure, 1,4,5,8-Naphthalintetracarbonsäure, 1,2,9,10-Decantetracarbonsäure, Benzophenontetracarbonsäure, 3,3',4,4'-Benzo-phenontetracarbonsäure, Tetrahydrofurantetracarbonsäure oder Cydopentantetracarbonsäuren wie Cyclopentan-1,2,3,4-tetracarbonsäure
zu nennen.

Ganz besonders bevorzugt werden gegebenenfalls mindestens einfach substituierte mono-, di-, tri-, tetra- oder höherkernige aromatische Di-, Tri- oder Tetracarbonsäuren eingesetzt, wobei jeder der Kerne mindestens ein Heteroatom enthalten kann, wobei zwei oder mehr Kerne gleiche oder unterschiedliche Heteroatome enthalten kann. Beispielsweise bevorzugt werden monokernige Dicarbonsäuren, monokernige Tricarbonsäuren, monokernige Tetracarbonsäuren, dikernige Dicarbonsäuren, dikernige Tricarbonsäuren, dikernige Tetracarbonsäuren, trikernige Dicarbonsäuren, trikernige Tricarbonsäuren, trikernige Tetracarbonsäuren, tetrakernige Dicarbonsäuren, tetrakernige Tricarbonsäuren und/oder tetrakernige Tetracarbonsäuren. Geeignete Heteroatome sind beispielsweise N, O, S, B, P, Si, bevorzugte Heteroatome sind hierbei N, S und/oder O. Als geeigneter Substituent ist diesbezüglich unter anderem -OH, eine Nitrogruppe, eine Aminogruppe oder eine Alkyl- oder Alkoxygruppe zu nennen.

Insbesondere bevorzugt werden als mindestens zweizähnige organische Verbindungen Acetylendicarbonsäure (ADC), Campherdicarbonsäure, Fumarsäure, Bernsteinsäure, Benzoldicarbonsäuren, Naphthalindicarbonsäuren, Biphenyldicarbonsäuren wie beispielsweise 4,4'-Biphenyldicarbonsäure (BPDC), Pyrazindicarbonsäuren, wie 2,5-Pyrazindicarbonsäure, Bipyridindicarbonsäuren wie beispielsweise 2,2'-Bipyridindicarbonsäuren wie beispielsweise 2,2'-Bipyndin-5,5'-dicarbonsäure, Benzoltricarbonsäuren wie beispielsweise 1,2,3-, 1,2,4-Benzoltricarbonsäure oder 1,3,5-Benzoltricarbonsäure (BTC), Benzoltetracarbonsäure, Adamantantetracarbonsäure (ATC), Adamantandibenzoat (ADB) Benzoltribenzoat (BTB), Methantetrabenzoat (MTB), Adamantantetrabenzoat oder Dihydroxyterephthalsäuren wie beispielsweise 2,5-Dihydroxyterephthalsäure (DHBDC) eingesetzt.

Ganz besonders bevorzugt werden unter anderem Isophtalsäure, Terephthalsäure, 2,5-Dihydroxyterephthalsäure, 1,2,3-Benzoltricarbonsäure, 1,3,5-Benzoltricarbonsäure, 2,6-Naphthalindicarbonsäure, 1,4-Naphthalindicarbonsäure, 1,2,3,4- und 1,2,4,5-Benzoltetracarbonsäure, Campherdicarbonsäure oder 2,2'-Bipyridin-5,5'-dicarbonsäure eingesetzt.

Neben diesen mindestens zweizähnigen organischen Verbindungen kann das metallorganische Gerüstmaterial auch einen oder mehrere einzähnige Liganden umfassen.

Vorzugsweise enthält die mindestens eine mindestens zweizähnige organische Verbindung keine Bor- oder Phosphoratome. Darüber hinaus enthält vorzugsweise das Gerüst des metallorganischen Gerüstmaterials keine Bor- oder Phosphoratome.

Das nicht-wässrige organische Lösemittel ist vorzugsweise ein C₁₋₆-Alkanol, Dimethylsulfoxid (DMSO), N,N-Dimethylformamid (DMF), N,N-Diethylformamid (DEF), Acetonitril, Toluol, Dioxan, Benzol, Chlorbenzol, Methylethylketon (MEK), Pyridin, Tetrahydrofuran (THF), Essigsäureethylester, gegebenenfalls halogeniertes C₁₋₂₀₀-Alkan, Sulfolan, Glykol, N-Methylpyrrolidon (NMP), gamma-Butyrolacton, alicyclische Alkohole wie Cyclohexanol, Ketone, wie Aceton oder Acetylaceton, Cycloketone, wie Cyclohexanon, Sulfolen oder Mischungen davon.

Ein C₁₋₆-Alkanol bezeichnet einen Alkohol mit 1 bis 6 C-Atomen. Beispiele hierfür sind Methanol, Ethanol, n-Propanol, i-Propanol, n-Butanol, i-Butanol, t-Butanol, Pentanol, Hexanol sowie Gemische davon.

Ein gegebenenfalls halogenisiertes C₁₋₂₀₀-Alkan bezeichnet ein Alkan mit 1 bis 200 C-Atomen, wobei ein oder mehrere bis hin zu allen Wasserstoffatomen durch Halogen, vorzugsweise Chlor oder Fluor, insbesondere Chlor, ersetzt sein kann bzw. können. Beispiele hierfür sind Chloroform, Dichlormethan, Tetrachlormethan, Dichlorethan, Hexan, Heptan, Oktan sowie Gemische davon.

Bevorzugte Lösemittel sind DMF, DEF und NMP. Besonders bevorzugt ist DMF.

Der Begriff "nicht-wässrig" bezieht sich vorzugsweise auf ein Lösemittel, das einen Höchstwassergehalt von 10 Gew.-%, mehr bevorzugt 5 Gew.-%, weiterhin mehr bevorzugt 1 Gew.-%, weiterhin bevorzugt 0,1 Gew.-%, besonders bevorzugt 0,01 Gew.-% bezogen auf das Gesamtgewicht des Lösemittels nicht überschreitet.

Vorzugsweise beträgt der Höchstwassergehalt während der Umsetzung 10 Gew.-%, mehr bevorzugt 5 Gew.-% und weiterhin mehr bevorzugt 1 Gew.-%.

Der Begriff "Lösemittel" betrifft reine Lösemittel sowie Gemische von unterschiedlichen Lösemitteln.

Weiterhin schließt sich an den Verfahrensschritt der Umsetzung der mindestens einen Metallverbindung mit der mindestens einen mindestens zweizähnigen organischen Verbindung ein Calcinierungsschritt an. Die hierbei eingestellte Temperatur beträgt mehr als 250°C, bevorzugt 300 bis 400 °C.

Aufgrund des Calcinierungsschrittes kann insbesondere bei der Herstellung von porösen Aluminiumgerüstmaterialien der in den Poren befindliche Ligand entfernt werden.

Ergänzend hierzu kann die Entfernung von Ligand aus den Poren des porösen metallorganischen Gerüstmaterials durch die Behandlung des gebildeten Gerüstmaterials mit einem nicht-wässrigen Lösemittel erfolgen. Hierbei wird in einer Art "Extraktionsverfahren" der Ligand entfernt und gegebenenfalls im Gerüstmaterial durch ein Lösemittelmolekül ersetzt. Diese schonende Methode ist insbesondere geeignet, wenn es sich bei dem Liganden um eine hochsiedende Verbindung handelt.

Die Behandlung erfolgt vorzugsweise mindestens 30 Minuten und kann typischerweise bis zu 2 Tagen durchgeführt werden. Dies kann bei Raumtemperatur oder erhöhter Temperatur geschehen. Vorzugsweise erfolgt dies unter erhöhter Temperatur, bei-B05/0587 spielsweise bei mindestens 40 °C, bevorzugt 60°C. Weiterhin bevorzugt erfolgt die Extraktion bei der Siedetemperatur des eingesetzten Lösemittels statt (unter Rückfluss).

Die Behandlung kann in einem einfachen Kessel durch Aufschlämmen und Rühren des Gerüstmaterials erfolgen. Es können auch Extraktionsapparaturen wie Soxhlet-Apparaturen, insbesondere technische Extraktionsapparaturen.

Als geeignete Lösemittel können die oben genannnten verwendet werden, also beispielsweise C₁₋₆-Alkanol, Dimethylsulfoxid (DMSO), N,N-Dimethylformamid (DMF), N,N-Diethylformamid (DEF), Acetonitril, Toluol, Dioxan, Benzol, Chlorbenzol, Methylethylketon (MEK), Pyridin, Tetrahydrofuran (THF), Essigsäureethylester, gegebenenfalls halogeniertes C₁₋₂₀₀-Alkan, Sulfolan, Glykol, N-Methylpyrrolidon (NMP), gamma-Butyrolacton, alicyclische Alkohole wie Cyclohexanol, Ketone, wie Aceton oder Acetylaceton, Cycloketone, wie Cyclohexanol oder Mischungen davon.

Bevorzugt sind Methanol, Ethanol, Propanol, Aceton, MEK und Mischungen davon.

Ein ganz besonders bevorzugtes Extraktionslösemittel ist Methanol.

Das verwendete Lösemittel zur Extraktion kann gleich oder verschieden zu demjenigen für die Umsetzung der mindestens einen Metallverbindung mit der mindestens einen mindestens zweizähnigen organischen Verbindung sein. Insbesondere ist es bei der "Extraktion" nicht unbedingt erforderlich aber bevorzugt, dass das Lösemittel wasserfrei ist.

Die metallorganischen Gerüstmaterialien gemäß der vorliegenden Erfindung enthalten Poren, insbesondere Mikro- und/oder Mesoporen. Mikroporen sind definiert als solche mit einem Durchmesser von 2 nm oder kleiner und Mesoporen sind definiert durch einen Durchmesser im Bereich von 2 bis 50 nm, jeweils entsprechend nach der Definition, wie sie Pure Applied Chem. 57 (1985), Seiten 603 bis 619, insbesondere auf Seite 606 angegeben ist. Die Anwesenheit von Mikro- und/oder Mesoporen kann mit Hilfe von Sorptionsmessungen überprüft werden, wobei diese Messungen die Aufnahmekapazität der MOF für Stickstoff bei 77 Kelvin gemäß DIN 66131 und/oder DIN 66134 bestimmt.

Die spezifische Oberfläche beträgt - berechnet nach dem Langmuir-Modell gemäß DIN 66135 (DIN 66131, 66134) für ein solches metallorganisches Gerüstmaterial in Pulverform bevorzugt mehr als 1000 m²/g und besonders bevorzugt mehr als 1250 m²/g.

MOF Formkörper können eine niedrigere spezifische Oberfläche besitzen; vorzugsweise jedoch mehr als 10 m²/g, mehr bevorzugt mehr als 50 m²/g, weiter mehr bevorzugt mehr als 500 m²/g.

Die Porengröße des metallorganischen Gerüstmaterials kann durch Wahl des geeigneten Liganden und/oder der mindestens zweizähnigen organischen Verbindung gesteuert werden. Häufig gilt, dass je größer die organische Verbindung desto größer die Porengröße ist. Vorzugsweise beträgt die Porengröße von 0,2 nm bis 30 nm, besonders bevorzugt liegt die Porengröße im Bereich von 0,3 nm bis 9 nm bezogen auf das kristalline Material.

In einem MOF-Formkörper treten jedoch auch größere Poren auf, deren Größenverteilung variieren kann. Vorzugsweise wird jedoch mehr als 50 % des gesamten Porenvolumens, insbesondere mehr als 75 %, von Poren mit einem Porendurchmesser von bis zu 1000 nm gebildet. Vorzugsweise wird jedoch ein Großteil des Porenvolumens von Poren aus zwei Durchmesserbereichen gebildet. Es ist daher weiter bevorzugt, wenn mehr als 25 % des gesamten Porenvolumens, insbesondere mehr als 50 % des gesamten Porenvolumens von Poren gebildet wird, die in einem Durchmesserbereich von 100 nm bis 800 nm liegen und wenn mehr als 15 % des gesamten Porenvolumens, insbesondere mehr als 25 % des gesamten Porenvolumens von Poren gebildet wird, die in einem Durchmesserbereich bis zu 10 nm liegen. Die Porenverteilung kann mittels Quecksilber-Porosimetrie bestimmt werden.

Das metallorganische Gerüstmaterial kann pulverförmig beziehungsweise als Agglomerate vorliegen. Das Gerüstmaterial kann als solches verwendet werden oder es wird in einen Formkörper umgewandelt. Bevorzugte Verfahren sind hierbei die Verstrangung oder Tablettierung. Bei der Formkörperherstellung kann das Gerüstmaterial weitere Materialien, wie beispielsweise Binder, Gleitmittel oder andere Additive aufweisen, welche während der Herstellung hinzugesetzt werden. Ebenso ist es denkbar, dass das Gerüstmaterial weitere Bestandteile aufweist, wie zum Beispiel Adsorbentien wie Aktivkohle oder dergleichen.

Hinsichtlich der möglichen Geometrien der Formkörper existieren im Wesentlichen keine Beschränkungen. Beispielsweise sind unter anderem Pellets wie beispielsweise scheibenförmige Pellets, Pillen, Kugeln, Granulat, Extrudate wie beispielsweise Stränge, Waben, Gitter oder Hohlkörper zu nennen.

Zur Herstellung dieser Formkörper sind grundsätzlich sämtliche geeigneten Verfahren möglich. Es sind insbesondere folgende Verfahrensführungen bevorzugt:
- Kneten/Kollern des Gerüstmaterials allein oder zusammen mit mindestens einem Bindemittel und/oder mindestens einem Anteigungsmittel und/oder mindestens
   einer Templatverbindung unter Erhalt zeine Gemisches; Verformen des erhaltenen Gemisches mittels mindestens einer geeigneten Methode wie beispielsweise Extrudieren; Optional Waschen und/oder Trocknen und/oder Calcinieren des Extrudates; Optional Konfektionieren.
- Aufbringen des Gerüstmaterials auf mindestens ein gegebenenfalls poröses Trägermaterial. Das erhaltene Material kann dann gemäß der vorstehend beschriebenen Methode zu einem Formkörper weiterverarbeitet werden.
- Aufbringen des Gerüstmaterials auf mindestens ein gegebenenfalls poröses Substrat.

Kneten/Kollem und Verformen kann gemäß eines jeden geeigneten Verfahrens erfolgen, wie beispielsweise in Ullmanns Enzyklopädie der Technischen Chemie, 4. Auflage, Band 2, S. 313 ff. (1972) beschrieben.

Beispielsweise kann das Kneten/Kollem und/oder Verformen mittels einer Kolbenpresse, Walzenpresse in Anwesenheit oder Abwesenheit mindestens eines Bindermaterials, Compoundieren, Pelletieren, Tablettieren, Extrudieren, Co-Extrudieren, Verschäumen, Verspinnen, Beschichten, Granulieren, bevorzugt Sprühgranulieren, Versprühen, Sprühtrocknen oder einer Kombination aus zwei oder mehr dieser Methoden erfolgten.

Ganz besonders werden Pellets und/oder Tabletten hergestellt.

Das Kneten und/oder Verformen kann bei erhöhten Temperaturen wie beispielsweise im Bereich von Raumtemperatur bis 300 °C und/oder bei erhöhtem Druck wie beispielsweise im Bereich von Normaldruck bis hin zu einigen hundert bar und/oder in einer Schutzgasatmosphäre wie beispielsweise in Anwesenheit mindestens eines Edelgases, Stickstoff oder einem Gemisch aus zwei oder mehr davon erfolgen.

Das Kneten und/oder Verformen wird gemäß einer weiteren Ausführungsform unter Zugabe mindestens eines Bindemittels durchgeführt, wobei als Bindemittel grundsätzlich jede chemische Verbindung eingesetzt werden kann, die die zum Kneten und/oder Verformen gewünschte Viskosität der zu verknetenden und/oder verformenden Masse gewährleistet. Demgemäß können Bindemittel im Sinne der vorliegenden Erfindung sowohl Viskositätserhöhende als auch Viskositätsemiedrigende Verbindungen sein.

Als unter anderem bevorzugte Bindemittel sind beispielsweise Aluminiumoxid oder Aluminiumoxid enthaltende Binder, wie sie beispielsweise in der WO 94/29408 beschrieben sind, Siliciumdioxid, wie es beispielsweise in der EP 0 592 050 A1 beschrieben ist, Mischungen ais Siliciumdioxid und Aluminiumoxid, wie sie beispielsweise in der WO 94/13584 beschrieben sind, Tonminerale, wie sie beispielsweise in der JP 03-037156 A beschrieben sind, beispielsweise Montmorillonit, Kaolin, Bentonit, Hallosit, Dickit, Nacrit und Anauxit, Alkoxysilane, wie sie beispielsweise in der EP 0 102 544 B1 beschrieben sind, beispielsweise Tetraalkoxysilane wie beispielsweise Tetramethoxysilan, Tetraethoxysilan, Tetrapropoxysilan, Tetrabutoxysilan, oder beispielsweise Trialkoxysilane wie beispielsweise Trimethoxysilan, Triethoxysilan, Tripropoxysilan, Tributoxysilan, Alkoxytitanate, beispielsweise Tetraalkoxytitanate wie beispielsweise Tetramethoxytitanat, Tetraethoxytitanat, Tetrapropoxytitanat, Tetrabutoxytitanat, oder beispielsweise Trialkoxytitanate wie beispielsweise Trimethoxytitanat, Triethoxytitanat, Tripropoxytitanat, Tributoxytitanat, Alkoxyzirkonate, beispielsweise Tetraalkoxyzirkonate wie beispielsweise Tetramethoxyzirkonat, Tetraethoxyzirkonat, Tetrapropoxyzirkonat, Tetrabutoxyzirkonat, oder beispielsweise Trialkoxyzirkonate wie beispielsweise Trimethoxyzirkonat, Triethoxyzirkonat, Tripropoxyzirkonat, Tributoxyzirkonat, Silikasole, amphiphile Substanzen und/oder Graphite zu nennen. Insbesondere bevorzugt ist Graphit

Als viskositätssteigernde Verbindung kann beispielsweise auch, gegebenenfalls zusätzlich zu den oben genannten Verbindungen, eine organische Verbindung und/oder ein hydrophiles Polymer wie beispielsweise Cellulose oder ein Cellulosederivat wie beispielsweise Methylcellulose und/oder ein Polyacrylat und/oder ein Polymethacrylat und/oder ein Polyvinylalkohol und/oder ein Polyvinylpyrrolidon und/oder ein Polyisobuten und/oder ein Polytetrahydrofuran und/oder ein Polyethylenoxid eingesetzt werden.

Als Anteigungsmittel kann unter anderem bevorzugt Wasser oder mindestens ein Alkohol wie beispielsweise ein Monoalkohol mit 1 bis 4 C-Atomen wie beispielsweise Methanol, Ethanol, n-Propanol, iso-Propanol, 1-Butanol, 2-Butanol, 2-Methyl-1-propanol oder 2-Methyl-2-propanol oder ein Gemisch aus Wasser und mindestens einem der genannten Alkohole oder ein mehrwertiger Alkohol wie beispielsweise ein Glykol, bevorzugt ein wassermischbarer mehrwertiger Alkohol, allein oder als Gemisch mit Wasser und/oder mindestens einem der genannten einwertigen Alkohole eingesetzt werden.

Weitere Additive, die zum Kneten und/oder Verformen eingesetzt werden können, sind unter anderem Amine oder Aminderivate wie beispielsweise Tetraalkylammonium-Verbindungen oder Aminoalkohole und Carbonat enthaltende Verbindungen wie etwa Calciumcarbonat. Solche weiteren Additive sind etwa in der EP 0 389 041 A1, der EP 0 200 260 A1 oder der WO 95/19222 beschrieben.

Die Reihenfolge der Additive wie Templatverbindung, Binder, Anteigungsmittel, viskositätssteigernde Substanz beim Verformen und Kneten ist grundsätzlich nicht kritisch.

Gemäß einer weiteren bevorzugten Ausführungsform wird der gemäß Kneten und/oder Verformen erhaltene Formkörper mindestens einer Trocknung unterzogen, die im Allgemeinen bei einer Temperatur im Bereich von 25 bis 300 °C, bevorzugt im Bereich von 50 bis 300 °C und besonders bevorzugt im Bereich von 100 bis 300 °C durchgeführt wird. Ebenso ist es möglich, im Vakuum oder unter Schutzgasatmosphäre oder durch Sprühtrocknung zu trocknen.

Gemäß einer besonders bevorzugten Ausführungsform wird im Rahmen dieses Trocknungsvorgangs mindestens eine der als Additive zugesetzten Verbindungen zumindest teilweise aus dem Formkörper entfernt.

Ein weiterer Gegenstand der vorliegenden Erfindung ist ein poröses metallorganisches gerüstmaterial erhältlich aus einem erfindungsgemäßen Verfahren zur dessen Herstellung. Hierbei weist das Gerüstmaterial vorzugsweise die weiter oben für Gerüstmaterialien im Allgemeinen angegeben spezifischen Oberflächen (nach Langmuir) auf.

Ein weiterer Gegenstand der vorliegenden Erfindung ist die Verwendung eines porösen metallorganischen Gerüstmaterials enthaltend mindestens ein Metall ausgewählt aus der Gruppe bestehend aus Be^{II}, Mg^{II}, Ca^{II}, Sr^{II}, Ba^{II}, Al^{III}, Ga^{III} und In^{III} und mindestens eine mindestens zweizähnige organische Verbindung, wobei die organische Verbindung zumindest zwei Atome jeweils unabhängig ausgewählt aus der Gruppe bestehend aus Sauerstoff, Schwefel und Stickstoff aufweist, über die die organische Verbindung an das Metall koordinativ bindet, insbesondere ein Gerüstmaterial erhältlich aus dem erfindungsgemäßen Verfahren, zur Aufnahme mindestens eines Stoffes zu dessen Speicherung, Abtrennung, kontrollierten Abgabe oder chemischen Umsetzung sowie als Trägermaterial, beispielsweise für Metalle, Metalloxide, Metallsulfide oder andere Gerüststrukturen.

Bei dem mindestens einen Stoff kann es sich um ein Gas oder eine Flüssigkeit handeln. Vorzugsweise handelt es sich bei dem Stoff um ein Gas.

Im Rahmen der vorliegenden Erfindung werden vereinfachend die Begriffe "Gas" und "Flüssigkeit" verwendet, wobei hier jedoch ebenso Gasgemische sowie Flüssigkeitsgemische beziehungsweise flüssige Lösungen unter dem Begriff "Gas" beziehungsweise "Flüssigkeit" zu verstehen sind.

Bevorzugte Gase sind Wasserstoff, Kohlenwasserstoffe, insbesondere Methan, Ethan, Ethen, Acetylen, Propan, n-Butari sowie i-Butan, Kohlenmonoxid, Kohlendioxid, Stickoxide, Sauerstoff, Schwefeloxide, Halogene, halogenierte Kohlenwasserstoffe, NF₃, SF₆, Ammoniak, Borane, Phosphane, Schwefelwasserstoff, Amine, Formaldehyd, Edelgase, insbesondere Helium, Neon, Argon, Krypton sowie Xenon.

Insbesondere bevorzugt ist die Verwendung eines erfindungsgemäßen metallorganischen Gerüstmaterials, bei dem das Metall Al^{III} oder Mg^{II} ist, zur Wasserstoffspeicherung.

Bei dem mindestens einen Stoff kann es sich jedoch auch um eine Flüssigkeit handeln. Beispiele für eine solche Flüssigkeit sind Desinfektionsmittel, anorganische oder organische Lösemittel, Treibstoffe - insbesondere Benzin oder Diesel -, Hydraulik-, Kühler-, Bremsflüssigkeit oder ein Öl, insbesondere Maschinenöl. Weiterhin kann es sich bei der Flüssigkeit um halogenierte aliphatische oder aromatische, cyclische oder acyclische Kohlenwasserstoffe oder Mischungen davon handeln. Insbesondere kann die Flüssigkeit Aceton, Acetonitril, Anilin, Anisol, Benzol, Benzonitril, Brombenzol, Butanol, tert.-Butanol, Chinolin, Chlorbenzol, Chloroform, Cyclohexan, Diethylenglykol, Diethylether, Dimethylacetamid, Dimethylformamid, Dimethylsulfoxid, Dioxan, Eisessig, Essigsäureanhydrid, Essigsäureethylester, Ethanol, Ethylencarbonat, Ethylendichlorid, Ethylenglykol, Ethylenglykoldimethylether, Formamid, Hexan, Isopropanol, Methanol, Methoxypropanol, 3-Methyl-1-butanol, Methylenchlorid, Methylethylketon, N-Methylformamid, N-Methylpyrrolidon, Nitrobenzol, Nitromethan, Piperidin, Propanol, Propylencarbonat, Pyrridin, Schwefelkohlenstoff, Sulfolan, Tetrachlorethen, Tetrachlorkohlenstoff, Tetrahydrofuran, Toluol, 1,1,1-Trichlorethan, Trichlorethylen, Triethylamin, Triethylenglykol, Triglyme, Wasser oder Mischungen hiervon handeln.

Weiterhin kann der mindestens eine Stoff ein Geruchsstoff sein.

Vorzugsweise handelt es sich bei dem Geruchsstoff um eine flüchtige organische oder anorganische Verbindung, die mindestens eines der Elemente Stickstoff, Phosphor, Sauerstoff, Schwefel, Fluor, Chlor, Brom oder lod enthält oder ein ungesättigter oder aromatischer Kohlenwasserstoff oder ein gesättigter oder ungesättigter Aldehyd oder ein Keton ist. Mehr bevorzugte Elemente sind Stickstoff, Sauerstoff, Phosphor, Schwefel, Chlor, Brom; insbesondere bevorzugt sind Stickstoff, Sauerstoff, Phosphor und Schwefel.

Insbesondere handelt es sich bei dem Geruchsstoff um Ammoniak, Schwefelwasserstoff, Schwefeloxide, Stickoxide, Ozon, cyclische oder acyclische Amine, Thiole, Thioether sowie Aldehyde, Ketone, Ester, Ether, Säuren oder Alkohole. Besonders bevorzugt sind Ammoniak, Schwefelwasserstoff, organische Säuren (bevorzugt Essigsäure, Propionsäure, Buttersäure, Isobuttersäure, Valeriansäure, Isovaleriansäure, Capronsäure, Heptylsäure, Laurinsäure, Pelargonsäure) sowie cyclische oder acyclische Kohlenwasserstoffe, die Stickstoff oder Schwefel enthalten sowie gesättigte oder ungesättigte Aldehyde, wie Hexanal, Heptanal, Oktanal, Nonanal, Decanal, Octenal oder Nonenal und insbesondere flüchtige Aldehyde wie Butyraldehyd, Propionaldehyd, Acetaldehyd und Formaldehyd und weiterhin Treibstoffe wie Benzin, Diesel (Inhaltsstoffe).

Bei den Geruchsstoffen kann es sich auch um Riechstoffe, die beispielsweise zur Herstellung von Parfümen verwendet werden, handeln. Beispielhaft seien als Riechstoffe oder Öle, die solche Riechstoffe freisetzen zu nennen: ätherische Öle, Basilikumöl, Geranienöl, Minzöl, Canangabaumöl, Kardamomöl, Lavendelöl, Pfefferminzöl, Muskatöl, Kamillenöl, Eukalyptusöl, Rosmarinöl, Zitronenöl, Limettenöl, Orangenöl, Bergamottenöl, Muskatellersalbeiöl, Korianderöl, Zypressenöl, 1,1-Dimethoxy-2-pherylethan, 2,4-Dimethyl-4-phenyltetrahydrofuran, Dimethyltetrahydrobenzaldehyd, 2,6-Dimethyl-7-octen-2-ol, 1,2-Diethoxy-3,7-dimethyl-2,6-octadien, Phenylacetaldehyd, Rosenoxid, Ethyl-2-methylpentanoat, 1-(2,6,6-Trimethyl-1,3-cydohexadien-1-yl)-2-buten-1-on, Ethylvanillin, 2,6-Dimethyl-2-octenol, 3,7-Dimethyl-2-octenol, tert-Butylcyclohexylacetat, Anisylacetate, Allylcyclohexyloxyacetat, Ethyllinalool, Eugenol, Cumarin, Ethylacetacetat, 4-Phenyl-2,4,6-trimethyl-1,3-dioxan, 4-Methylen-3,5,6,6-tetramethyl-2-heptanon, Ethyltetrahydrosafranat, Geranylnitril, cis-3-Hexen-1-ol, cis-3-Hexenylacetat, cis-3-Hexenylmethylcarbonate, 2,6-Dimethyl-5-hepten-1-al, 4-(Tricydo[5.2.1.0]decylidene)-8-butanal, 5-(2,2,3-Trimethyl-3-cyclopentenyl)-3-methylpentan-2-ol, p-tert-Butylalpha-methylhydrozimtaldehyd, Ethyl[5.2.1.0]tricydodecancarboxylat, Geraniol, Citronellol, Citral, Linalool, Linalylacetat, Jonone, Phenylethanol oder Mischungen hiervon.

Im Rahmen der vorliegenden Erfindung weist ein flüchtige Geruchsstoff vorzugsweise einen Siedepunkt oder Siedepunktsbereich von weniger als 300 °C auf. Mehr bevorzugt ist der Geruchsstoff eine leicht flüchtige Verbindung oder Gemisch. Insbesondere bevorzugt weist der Geruchsstoff einen Siedepunkt oder Siedebereich von weniger als 250 °C, mehr bevorzugt weniger als 230 °C, insbesondere bevorzugt weniger als 200 °C auf.

Bevorzugt sind ebenfalls Geruchsstoffe, die eine hohe Flüchtigkeit aufweisen. Als Maß für die Flüchtigkeit kann der Dampfdruck herangezogen werden. Im Rahmen der vorliegenden Erfindung weist ein flüchtiger Geruchsstoff vorzugsweise einen Dampfdruck von mehr als 0,001 kPa (20°C) auf. Mehr bevorzugt ist der Geruchsstoff eine leicht flüchtige Verbindung oder Gemisch. Insbesondere bevorzugt weist der Geruchsstoff einen Dampfdruck von mehr als 0,01 kPa (20°C) auf, mehr bevorzugt einen Dampfdruck von mehr als 0,05 kPa (20°C) auf. Besonders bevorzugt weisen die Geruchsstoffe einen Dampfdruck von mehr als 0,1 kPa (20°C) auf.

### Beispiele

### Beispiel 1 Drucklose Herstellung eines AI-BDC-MOFs in DMF

5,8 g Terephthalsäure (BDC) und 26 g Al(NO₃)₃*9H₂O werden in 100 ml DMF suspendiert und die Mischung 16 h unter Rückfluss bei 133 °C gerührt. Anschließend wird der Feststoff abfiltriert, mit 20 ml Methanol gewaschen und in einem Vakuumtrockenschrank bei 200°C 16 h lang getrocknet. Abschließend wird das Pulver 3 Tage lang in einem Muffelofen (100 I/h Luft) bei 330 °C nachbehandelt (Aufheizen mit ca. 75 °C/h).

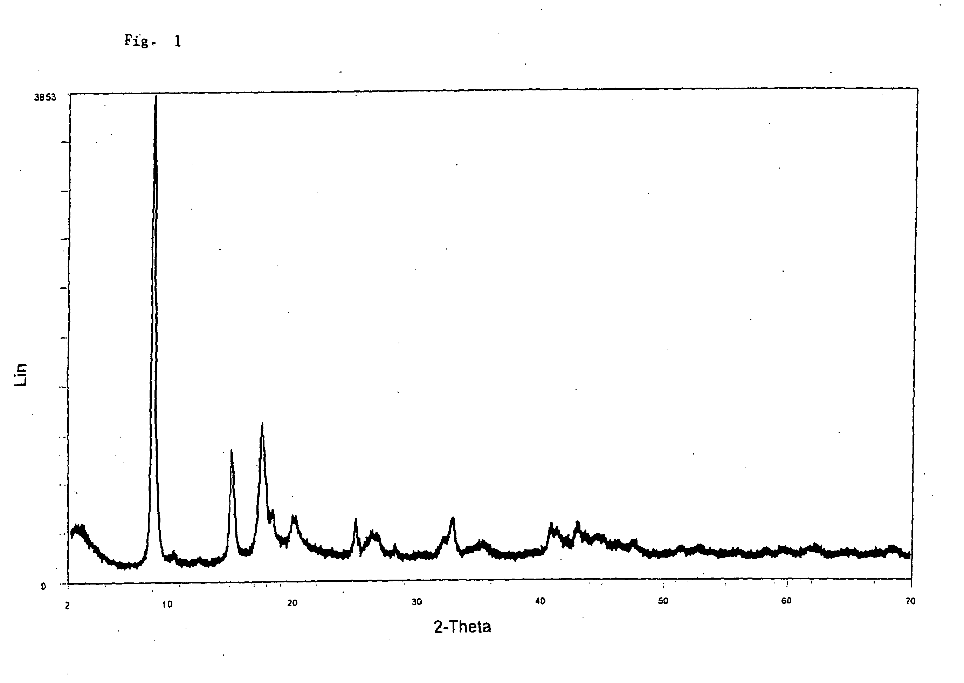

Es werden 7,7 g eines hellbraunen Pulvers erhalten mit einer Oberfläche von 1267 m²/g (nach Langmuir). Das Diffraktogramm (XRD) ist in Fig. 1 dargestellt. Für sämtlich Diffraktogramme wird die Probe ungemörsert unter einer N₂-Glocke präpariert und mit einer Styroflexfolie luftdicht überzogen. Die Probe wird an einem Gerät D5000 der Firma Siemens mit einer Cu-Anode bei einer Schrittbreite von 0,02° und einer Schrittgeschwindigkeit von 3,6 s aufgenommen.

### Beispiel 2 Drucklose Herstellung eines Al-BDC-MOFs in DMF

19,9 g Terephthalsäure (BDC) und 23,3 g Al₂(SO₄)₃*18H₂O werden in 100 ml DMF suspendiert und die Mischung 16 h unter Rückfluss bei 130 °C gerührt (350 U/min). Anschließend wird der Feststoff abfiltriert, mit 3 x 20 ml DMF und 20 ml Methanol gewaschen und in einem Vakuumtrockenschrank bei 200°C 16 h lang getrocknet. Abschließend wird das Pulver 3 Tage lang in einem Muffelofen (100 I/h Luft) bei 330 °C nachbehandelt (Aufheizen mit ca. 75 °C/h).

Es werden 15,7 g Produkt erhalten mit einer Oberfläche von 1398 m²/g (nach Langmuir).

### Beispiel 3 Drucklose Herstellung eines Al-BDC-MOFs in DEF

19,9 g Terephthalsäure (BDC) und 23,3g Al₂(SO₄)₃*18H₂O werden in 100 ml DEF suspendiert und die Mischung 16 h bei einer Außentemperatur des Ölbades von 130 °C gerührt (290 U/min). Der Ansatz ruht zunächst ca. 60 h bei Raumtemperatur. Anschließend wird der Feststoff abfiltriert, mit 7 x 20 ml DMF und 2 x 20 ml Methanol gewaschen und in einem Vakuumtrockenschrank bei 200°C 16 h lang getrocknet. Abschließend wird das Pulver 3 Tage lang in einem Muffelofen (100 l/h Luft) bei 330 °C nachbehandelt (Aufheizen mit ca. 75 °C/h).

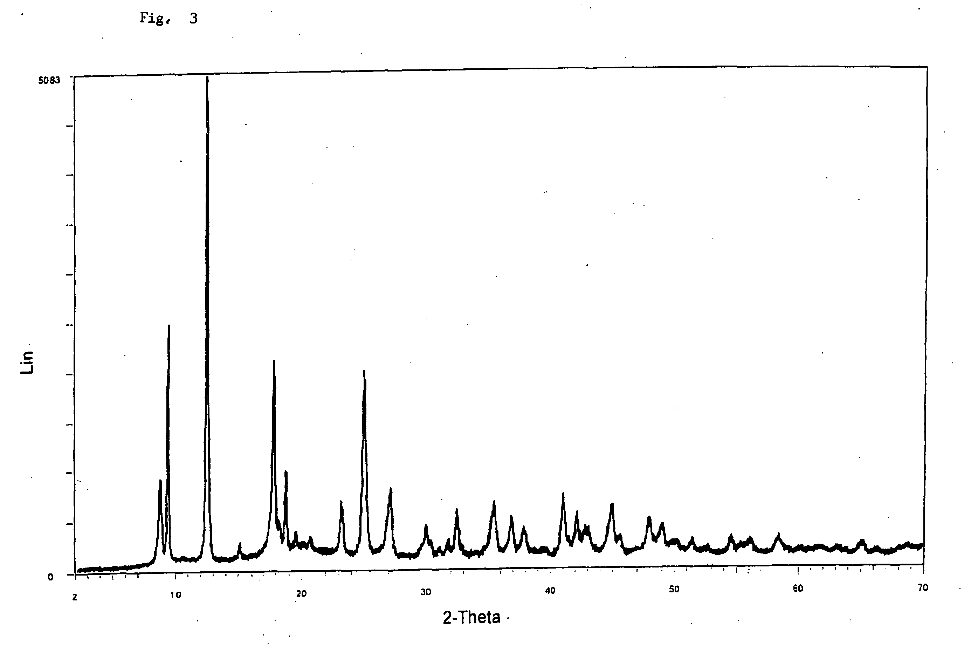

Es werden 16 g Produkt erhalten mit einer Oberfläche von 1425 m²/g (nach Langmuir). Das Diffraktogramm (XRD) ist in Fig. 3 dargestellt.

### Vergleichsbeispiel 4 Drucklose Herstellung eines Al-BDC-MOFs in Wasser

5,8 g Terephthalsäure (BDC) und 23,3 g Al₂(SO₄)₃*18H₂O werden in 100 ml Wasser suspendiert und die Mischung 3 Tage unter Rückfluss bei 100 °C gerührt (500 U/min). Der Ansatz ruht zunächst ca. 60 h bei Raumtemperatur. Anschließend wird der Feststoff abfiltriert, mit 3 x 20 ml Wasser und 3 x 20 ml Methanol gewaschen und in einem Vakuumtrockenschrank bei 200°C 16 h lang getrocknet. Abschließend wird das Pulver 3 Tage lang in einem Muffelofen (100 l/h Luft) bei 330 °C nachbehandelt (Aufheizen mit ca. 75 °C/h).

Es werden lediglich 1,5 g Produkt erhalten mit einer Oberfläche von 1416 m²/g (nach Langmuir).

### Vergleichsbeispiel 5 Herstellung eines Al-BDC-MOFs in Wasser unter Druck und ohne Nachbehandlung

5,76 g Terephthalsäure (BDC) und 26 g Al(NO₃)₃*9H₂O werden in 100 ml Wasser suspendiert und die Mischung bei Umgebungsbedingungen 30 min verrührt. Anschließend wird der Ansatz in einem Berghoff-Autoklaven ("Teflon-Liner") 3 Tage bei 220 °C gehalten. Nach Filtrieren wird mit 5 x 20 ml Wasser gewaschen und 3 Tage lang in Muffelofen (100 I/h Luft) bei 330 °C getrocknet.

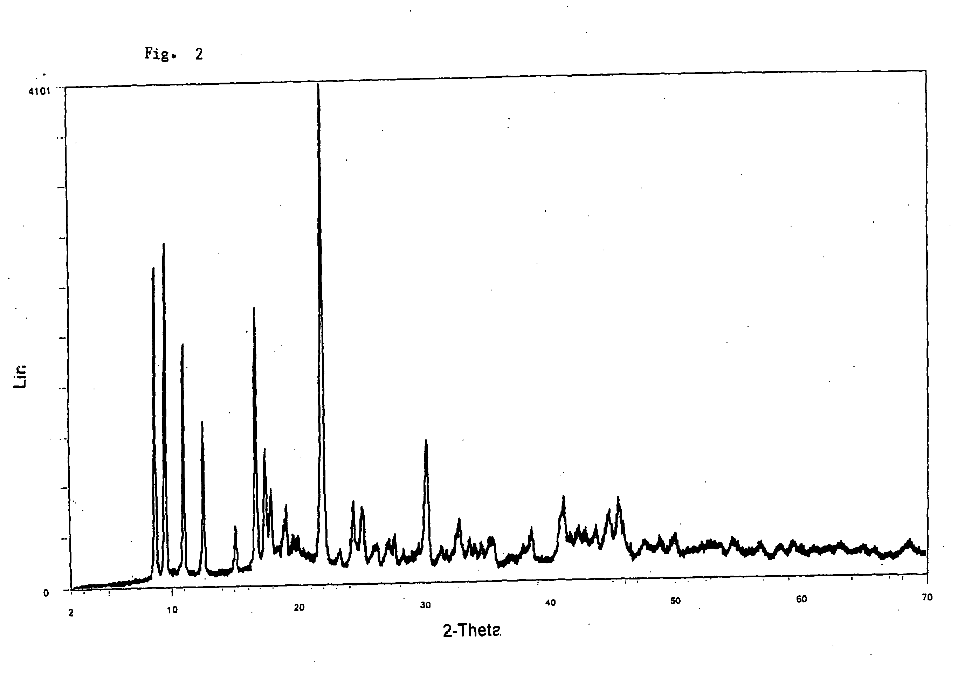

Es werden lediglich 4,6 g Produkt erhalten mit einer Oberfläche von 319 m²/g (nach Langmuir). Das Diffraktogramm (XRD) ist in Fig. 2 dargestellt.

### Vergleichsbeispiel 6 Herstellung eines Al-BDC-MOFs in DMF unter Druck

5,8 g Terephthalsäure (BDC) und 26 g Al(NO₃)₃*9H₂O werden in 100 ml DMF suspendiert und die Mischung bei Umgebungsbedingungen 10 min verrührt. Anschließend wird der Ansatz in einem Berghoff-Autoklaven ("Teflon-Liner") 1 Tage bei 170 °C gehalten. Nach Filtrieren wird mehrmals mit DMF und anschließend mehrmals mit Aceton gewaschen. Das Produkt wurde zunächst im Vakuumtrockenschrank bei 130°C 20 h vorgetrocknet und 3 Tage lang in einem Muffelofen (100 I/h Luft) bei 330 °C getrocknet.

Es werden 7,8 g Produkt erhalten mit einer Oberfläche von 1196 m²/g (nach Langmuir). Ein Scale up konnte nicht durchgeführt werden.

### Beispiel 7 Drucklose Herstellung eines Al-BDC-MOFs in DMF (Scale up)

292,9 g Terephthalsäure (BDC) und 250,1 g Al₂(SO₄)₃*18H₂O werden in 1257 g DMF suspendiert und unter Rühren 24 h auf 130 °C erwärmt. Anschließend wird die Suspension filtriert und der Filterkuchen mehrmals mit DMF nachgewaschen. Der Filterkuchen wird zunächst zwei Tage an Luft vorgetrocknet und anschließend 72 h bei 320 °C in einem Muffelofen unter Luftatmosphäre getrocknet.

Es werden 192 g Produkt erhalten mit einer Oberfläche von 1483 m²/g (nach Langmuir).

### Beispiel 8 Drucklose Herstellung eines Al-BDC-MOFs

19,9 g Terephthalsäure (BDC) und 23,3 g Al₂(SO₄)₃*18H₂O werden in 100 g NMP suspendiert und unter Rühren 24 h auf 130 °C erwärmt. Anschließend wird die Suspension filtriert und der Filterkuchen mehrmals mit NMP nachgewaschen. Der Filterkuchen wird zunächst zwei Tage an Luft vorgetrocknet und anschließend 72 h bei 320 °C in einem Muffelofen unter Luftatmosphäre getrocknet.

Es werden 15,1 g Produkt erhalten mit einer Oberfläche von 1134 m²/g (nach Langmuir).

### Beispiel 9 Isobuten-Polymerisation mit Hilfe des Gerüstmaterials aus Beispiel 3

5 g Gerüstmaterial werden in einem 300 ml Stahlautoklaven vorgelegt und mit 100 g Toluol versetzt. In den verschlossenen Autoklaven werden 82 ml flüssiges Isobuten eingepresst. Der Autoklav wurde 3 h bei 170 °C gehalten. Nach Abkühlen wird die Flüssigphase im GC analysiert (GC-Fl%): Enthalten sind u.a. 68 % C₄, 26 % C₈. Daneben werden auch höhere Olefine zum Beispiel 2,3 % C₁₆ und 1,7 % C₂₀ gefunden.

### Beispiel 10 Speicherung von H₂ mit Hilfe des Gerüstmaterials aus Beispiel 7

Der Al-MOF aus Beispiel 7 wird vor der Messung bei einem Druck kleiner 1 mbar bei 80°C 5 Stunden evakuiert. 161,96 g des getrockneten Produkts werden in einen leeren Behälten überführt. Dieser wird anschließend auf 77 K abgekühlt und evakuiert. Über eine Gasdosierungseinrichtung werden portionsweise Wasserstoff zugegeben und jeweils die aufgenommene Menge gravimetrisch bestimmt. Bei 50,3 bar erreicht der Al-MOF eine Wasserstoffaufnahme von etwa 3 wt%.

### Beispiel 11 Speicherung von Methan mit Hilfe des Gerüstmaterials aus Beispiel 7

Der Al-MOF aus Beispiel 7 wird vor der Messung bei einem Druck kleiner 1 mbar bei 80°C 5 Stunden evakuiert. 161,96 g des getrockneten Produkts werden in einen leeren Behälter überführt. Dieser wird anschließend auf 25°C temperiert und evakuiert. Über eine Gasdosierungseinrichtung wurde portionsweise Methan zugegeben und jeweils die aufgenommene Menge gravimetrisch bestimmt. Bei 50,4 bar können so in der MOF-gefüllten Flasche 40,3 g/L, bei 99,7 bar sogar 79,8 g/L Methan gespeichert werden

### Vergleichsbeispiel 12 Drucklose Herstellung eines Al-BDC-MOFs in DMF (Scale up)

398,7 g Terephthalsäure (BDC) und 466,5 g Al₂(SO₄)₃*18H₂O werden in 2000 ml DMF suspendiert und unter Rühren 24 h auf 115 °C erwärmt. Anschließend wird die Suspension filtriert und der Filterkuchen mehrmals mit 3x 400 ml DMF und 400 ml Methanol nachgewaschen. Der Filterkuchen wird zunächst mit N₂ während 96 h trockengeblasen. Es wurden 375 g Produkt erhalten.
15 g des getrockneten Produkts werden in einem Soxhlet-Extraktor 48 h mit Methanol extrahiert und abfiltriert. Der Filterkuchen wurde mit 3x25 ml Methanol nachgewaschen und 24 h bei 110°C im Vakuumtrockenschrank getrocknet.

Die Oberfläche des extrahierten Materials betrug 1522 m²/g (nach Langmuir).

### Beispiel 13 Drucklose Herstellung eines Al-BTC-MOFs in DMF

7,8 g Benzoltricarbonsäure (BTC) und 22,9 g Al(NO₃)₃*9H₂O werden in 520,5 g DMF suspendiert und unter Rühren 4 Tage auf 130 °C erwärmt. Anschließend wird die Suspension filtriert und der Filterkuchen mit 2x 100 ml DMF und 4x100 ml Methanol nachgewaschen. Der Filterkuchen wird zunächst im Vakuumtrockenschrank bei 200°C 16 h getrocknet. Abschließend wird das Pulver 3 Tage lang in einem Muffelofen (100 1/h Luft) bei 330 °C nachbehandelt (Aufheizen mit ca. 75 °C/h).

Es werden 8,4 g Produkt erhalten mit einer Oberfläche von 1791 m²/g (nach Langmuir).

### Beispiel 14 Drucklose Herstellung eines AI-BTC-MOFs in DMF

7,8 g Benzoltricarbonsäure (BTC) und 14,7 g AlCl₃*6H₂O werden in 520,5 g DMF suspendiert und unter Rühren 4 Tage auf 130 °C erwärmt. Anschließend wird die Suspension filtriert und der Filterkuchen mit 2x 100 ml DMF und 4x100 ml Methanol nachgewaschen. Der Filterkuchen wird zunächst im Vakuumtrockenschrank bei 200°C 16 h getrocknet. Abschließend wird das Pulver 3 Tage lang in einem Muffelofen (100 I/h Luft) bei 330 °C nachbehandelt (Aufheizen mit ca. 75 °C/h).

Es werden 10,9 g Produkt erhalten mit einer Oberfläche von 1451 m²/g (nach Langmuir).

### Vergleichsbeispiel 15 Drucklose Herstellung eines Al-BTC-MOFs in Wasser

7,8 g Benzoltricarbonsäure (BTC) und 22,9 g Al(NO₃)₃*9H₂O werden in 130,8 g Wasser suspendiert und unter Rühren 4 Tage auf 100 °C erwärmt. Anschließend wird die Suspension filtriert und der Filterkuchen mit Wasser nachgewaschen. Der Filterkuchen wird im Vakuumtrockenschrank bei 150°C 2 Tagen getrocknet.

Es werden lediglich 0,6 g Produkt erhalten.

### Vergleichsbeispiel 16 Hydrothermale Herstellung eines Al-BTC-MOFs in H₂O

7,8 g Benzoltricarbonsäure (BTC) und 22,9 g AI(NO₃)₃*9H₂O werden in 130,8 g Wasser suspendiert und in einem Berghoff-Autoklaven (Teflon-Liner) 4 Tage auf 130 °C erwärmt. Anschließend wird die Suspension filtriert und der Filterkuchen mit Wasser nachgewaschen. Der Filterkuchen wird im Vakuumtrockenschrank bei 150°C 2 Tagen getrocknet.

Es werden lediglich 0,4 g Produkt erhalten.

### Beispiel 17/Beispiel 18 Herstellung eines Al-BDC-MOFs in DMF

250,1 g Terephthalsäure (BDC) und 292,9 g Al₂(SO₄)₃*18H₂O werden in 1257 g DMF suspendiert und unter Rühren für 24 h auf 130 °C erwärmt. Anschließend wird die Suspension filtriert und der Filterkuchen mit DMF nachgewaschen. Der Filterkuchen wird 2 h bei 120°C im Trockenschrank getrocknet.

Anschließend wird die eine Hälfte bei 250°C im Trockenschrank 24 h lang calciniert. Der Gewichtsverlust beträgt 38,6%. Es entsteht ein Produkt mit einer Oberfläche von 634 m²/g.

Die andere Hälfte wird bei 320°C im Muffelofen während 3 h calciniert. Der Gewichtsverlust beträgt 59,8%. Es entsteht ein Produkt mit einer Oberfläche von 1368 m²/g.

### Vergleichsbeispiel 19 Drucklose Herstellung eines Al-Campherdisäure-MOFS in DEF

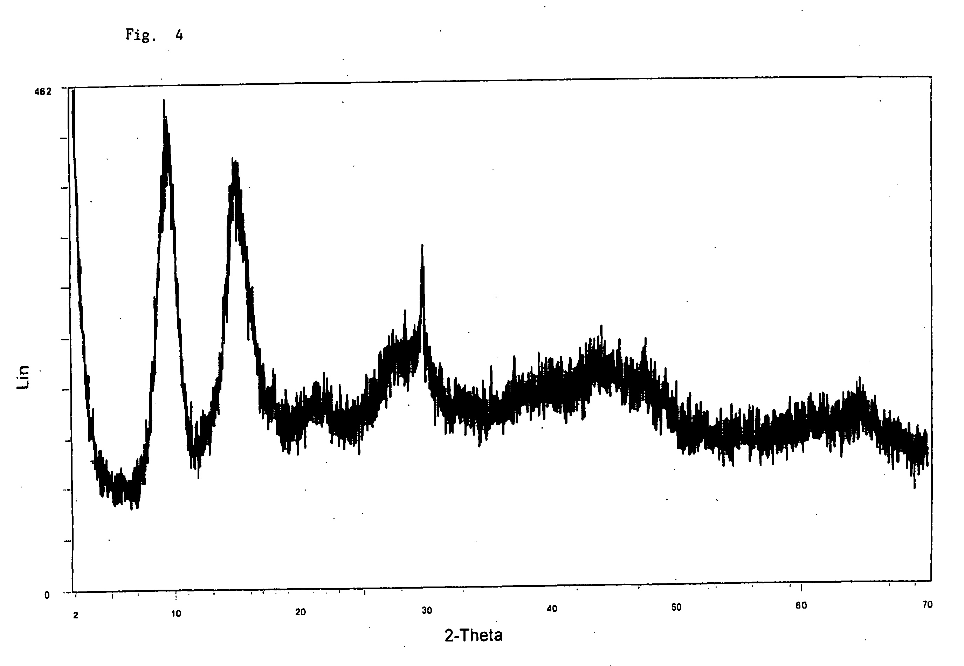

7,4g (+)-Campherdisäure und 24 g Al(SO₄)₃*18H₂O werden in 520,5 g DEF suspendiert und unter Rühren 4 Tage auf 130 °C erwärmt. Anschließend wird die Suspension filtriert und der Filterkuchen mit 2x 100 ml DEF und 4x100 ml Methanol nachgewaschen. Der Filterkuchen wird im Vakuumtrockenschrank bei 200°C 16 h getrocknet. Es fallen 11,8 g eines Produkts mit einer Oberfläche von 429 m²/g (nach Langmuir) an. Das Diffraktogramm (XRD) ist in Fig. 4 dargestellt. 1,5 g des Produktes werden in siedendem Methanol in einer Soxhlet-Apparatur 16 h extrahiert und anschließend bei Raumtemperatur im Vakuum getrocknet. Die Oberfläche verbessert sich auf 555 m²/g.

### Vergleichsbeispiel 20 Drucklose Herstellung eines Al-Butantetracarbonsäure-MOFs in DEF

8,7g 1,2,3,4-Butantetracarbonsäure und 48 g Al(SO₄)₃*18H₂O werden in 520,5 g DEF suspendiert und unter Rühren 4 Tage auf 130 °C erwärmt. Anschließend wird die Suspension filtriert und der Filterkuchen mit 2x 100 ml DEF und 4x100 ml Methanol nachgewaschen. Der Filterkuchen wird im Vakuumtrockenschrank bei 200°C 16 h getrocknet. Es fallen 14,9 g eines Produkts mit einer Oberfläche von 518 m²/g (nach Langmuir) an.

### Vergleichsbeispiel 21 DrUck/ase Herstellung eines A/-Mercaptabernsteinsäure-MOFs in DEF

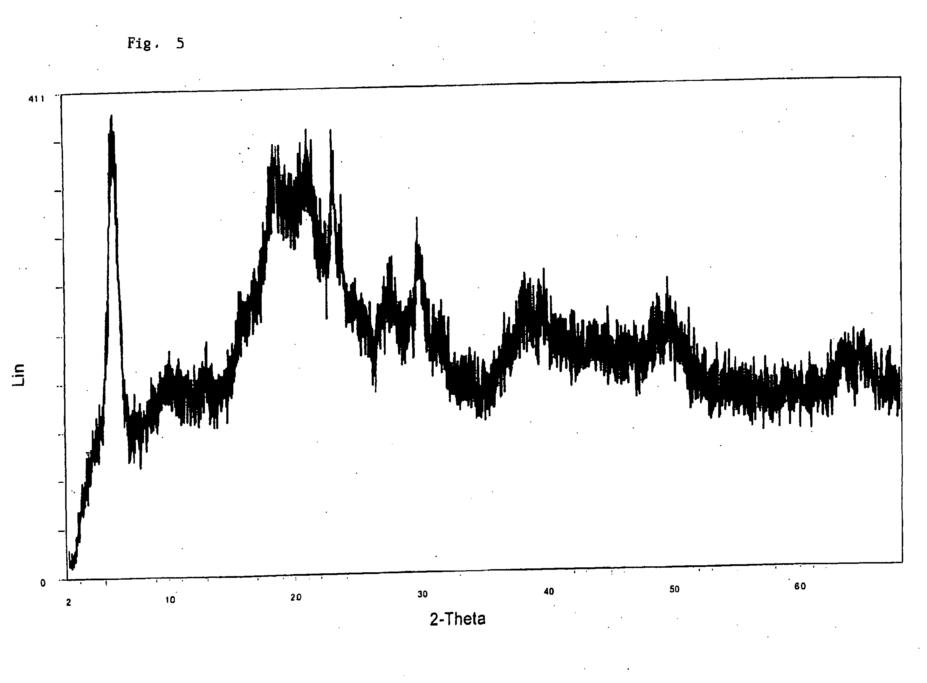

5,6 g Mercaptobernsteinsäure und 27,8 g Al(NO₃)₃*9H₂O werden in 520,5 g DEF suspendiert und unter Rühren 3 Tage auf 130 °C erwärmt. Anschließend wird die Suspension filtriert und der Filterkuchen mit 2x 100 ml DEF und 4x100 ml Methanol nachgewaschen. Der Filterkuchen wird im Vakuumtrockenschrank bei 200°C 16 h getrocknet. Es fallen 7,8 g eines Produkts mit einer Oberfläche von 488 m²/g (nach Langmuir) an. Das Diffraktogramm (XRD) ist in Fig. 5 dargestellt.

### Vergleichsbeispiel 22 Drucklose Herstellung eines Al-1,4-Naphthalindicarbonsäure-MOFs in DMF

8 g 1,4-Naphthalindicarbonsäure und 27,8 g Al(NO₃)₃*9H₂O werden in 520,5 g DMF suspendiert und unter Rühren 3 Tage auf 130 °C erwärmt. Anschließend wird die Suspension filtriert und der Filterkuchen mit 3x100 ml Methanol nachgewaschen. Der Filterkuchen wird im Vakuumtrockenschrank bei 200°C 16 h getrocknet. Es fallen 8,7 g eines Produkts an. Anschließend werden 1,5 g in einem Soxhlet-Extraktor 16 h mit Methanol extrahiert und 16 h bei Raumtemperatur im Vakuum getrocknet.

Es ergibt sich eine spezifische Oberfläche von 752 m²/g.

### Beispiel 23 Drucklose Herstellung eines Mg-2,6-Naphthalindicarbonsäure-MOFs in DEF

4,9 g 2,6-Naphthalindicarbonsäure und 8,16 g Mg(NO₃)₂*6H₂O werden in 1686 g DEF suspendiert und unter Rühren 24 h auf 105 °C erwärmt. Anschließend wird die Suspension filtriert und der Filterkuchen zunächst mit DMF und anschließend mit Chloroform nachgewaschen. Der Filterkuchen wird an Luft getrocknet. Es fallen 4,8 g eines Produkts an. Das getrocknete Produkt wird 2 Tage bei 330°C im Muffelofen calciniert.

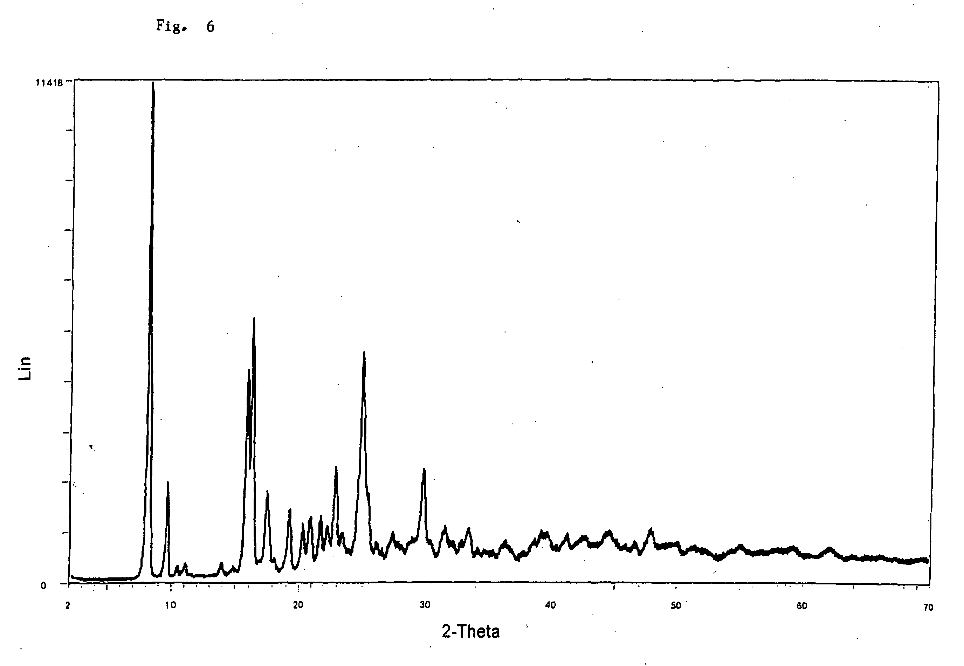

Es fallen 3,6 g Produkt an. Das Diffraktogramm (XRD) ist in Fig. 6 dargestellt. Es handelt sich um eine kristalline Substanz mit einem für MOFs typischen Diffraktogramm.

### Beispiel 24 Herstellung eines Al-1,2,4,5-Benzoltetracarbonsäure-MOFS

9,4 g 1,2,4,5-Benzoltetracarbonsäure und 30,5 g Al(NO₃)₃*9H₂O werden in 520,5 g DMF in einem 1 I-Rührkolben suspendiert, auf 130°C erwärmt und unter Rühren 72 h bei diesen Bedingungen gehalten. Das ausgefallene Produkt wird abfiltriert, mit 2 x 100 ml DMF und 4 x 100 ml Methanol gewaschen und im Vakuumtrockenschrank 16 h bei 200°C getrocknet. Abschließend wird das Produkt 72 h in einem Muffelofen unter Luftatmosphäre bei 330°C kalziniert.

Es werden 12,8 g Produkt mit einer N₂-Oberfläche von 930 m²/g (nach Langmuir) erhalten.

### Vergleichsbeispiel 25 Herstellung eines Al-Mercaptobemsteinsäure-MOFs

13,6 g Mercaptobernsteinsäure und 67,6 g Al(NO₃)₃*9H₂O werden in 1246 g DMF in einem 2 1-Rührkolben suspendiert, auf 130°C erwärmt und unter Rühren 72 h bei diesen Bedingungen gehalten. Das ausgefallene Produkt wird abfiltriert, mit 1 x 200 ml DMF und 3 x 200 ml Methanol gewaschen und im Vakuumtrockenschrank 16 h bei 200°C getrocknet.

Es werden 17,7 g eines gelblichen Produkts mit einer N₂-Oberfläche von 551 m²/g (nach Langmuir) erhalten.

### Beispiel 26 Herstellung eines Al-Terephthalsäure-MOFs

239 kg Terephthalsäure und 279 kg Al₂(SO₄)₃*18H₂O werden in 1500 kg DMF suspendiert, auf 130°C erwärmt und unter Rühren 18 h bei diesen Bedingungen gehalten. Das ausgefallene Produkt wird abfiltriert, mit 3 x 500 1 Aceton gewaschen. Anschließend wird der Filterkuchen zweimal in 500 1 Methanol bei 60°C für 3 h resuspendiert und das Methanol anschließend wieder abfiltriert. Der Filterkuchen wird anschließend im Vakuum bei anfänglich 50°C, später 100°C getrocknet.

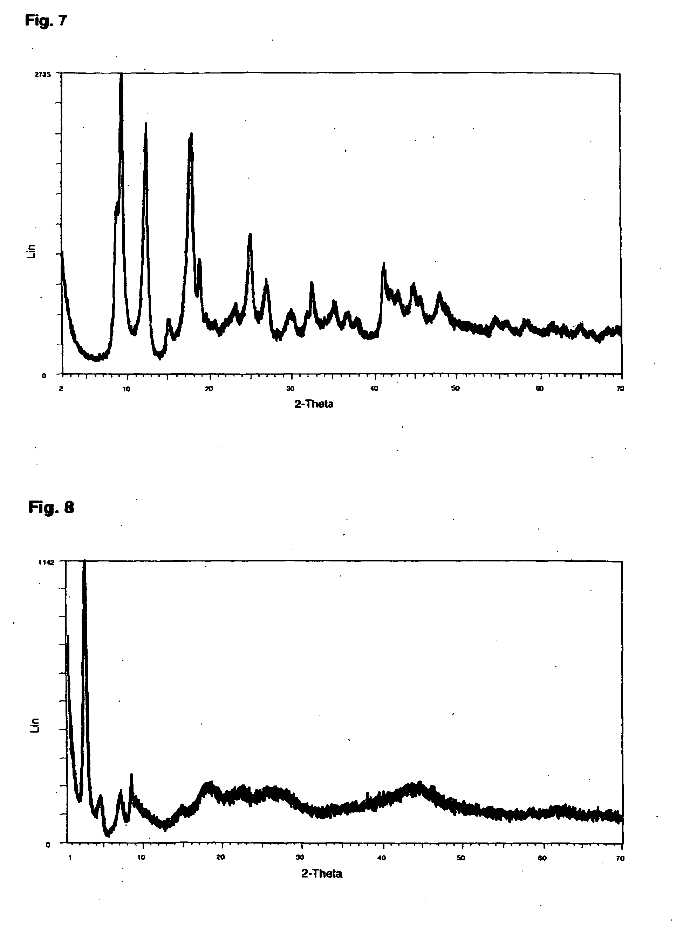

Es werden 249 kg eines weißen Materials erhalten. Nach einer Nachbehandlung im Muffelofen bei 360°C für 48 h unter Luftatmosphäre weist das Material eine N₂-Oberfläche von 1400 m²/g auf. Das XRD ist in Fig. 7 dargestellt.

### Vergleichsbeispiel 27 Herstellung eines Al-Isophthatsäure-MOFs

7,23 g Isophthalsäure und 7,0 g AlCl₃*6H₂O werden in 300 ml DMF in einem Rührkolben suspendiert, auf 130°C erwärmt und unter Rühren 20,5 h bei diesen Bedingungen gehalten. Das ausgefallene Produkt wird abfiltriert, mit 3 x 50 ml DMF und 4 x 50 ml Methanol gewaschen und im Vakuumtrockenschrank 16 h bei 110°C getrocknet.

Es werden 3,94 g eines gelben Produkts mit einer N₂-Oberfläche von 1242 m²/g (nach Langmuir; Aktivierungstemperatur 150°C) erhalten. Laut Elementaranalyse enthält das Material 42,8% C, 8,8% Al und 4,5% H. Das XRD ist in Fig. 8 gezeigt.

### Vergleichsbeispiel 28 Herstellung eines Al-4,5-Imidazoldicarbonsäure-MOFs

5,17 g 4,5-Imidazoldicarbonsäure und 5,33 g AlCl₃*6H₂O werden in 300 ml DMF in einem Rührkolben suspendiert, auf 130°C erwärmt und unter Rühren 20,5 h bei diesen Bedingungen gehalten. Das ausgefallene Produkt wird abfiltriert, mit 3 x 50 ml DMF und 4 x 50 ml Methanol gewaschen und im Vakuumtrockenschrank 72 h bei 110°C getrocknet.

Es werden 3,94 g eines schwach orangen Produkts mit einer N₂-Oberfläche von 703 m²/g (nach Langmuir; Aktivierungstemperatur 150°C) erhalten. Laut Elementaranalyse enthält das Material 35,3% C, 8,5% Al und 3,7% H.

### Vergleichsbeispiel 29 Herstellung eines Al-Cyclohexandicarbonsäure-M0Fs

1,33 g Cyclohexandicarbonsäure (cis/trans-Gemisch) und 5,95 g Al(NO₃)₃*9H₂O werden in 111 g DMF in einem Rührkolben suspendiert, auf 130°C erwärmt und unter Rühren 17 h bei diesen Bedingungen gehalten. Das ausgefallene Produkt wird abfiltriert, mit 2 x 50 ml DMF und 4 x 50 ml Methanol gewaschen und im Vakuumtrockenschrank 16 h bei 130°C getrocknet.

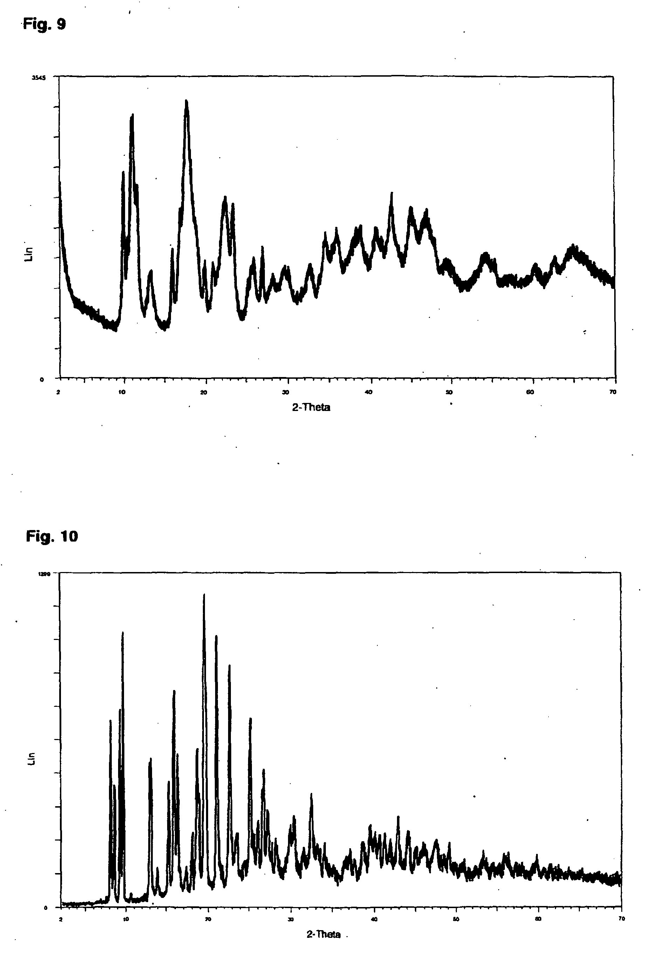

Es werden 3,94 g eines weißen Produkts mit einer N₂-Oberfläche von 410 m²/g (nach Langmuir) erhalten. Das XRD ist in Fig. 9 gezeigt.

### Vergleichsbeispiel 30 Herstellung eines Mg-Naphthalindicarbonsäure-MOFs

6,66 g 2,6-Naphthalindicarbonsäure und 10,98 g Mg(NO₃)₂*6H₂O werden in 137 g DMF in einem Rührkolben suspendiert, auf 130°C erwärmt und unter Rühren 48 h bei diesen

Bedingungen gehalten. Das in der Reaktion entstehende Wasser wird mit einem N₂-Strom über eine Destillationsbrücke ausgetrieben. Das ausgefallene Produkt wird abfiltriert, 2 mal mit DMF und 2 mal mit Chloroform gewaschen. Der Filterkuchen wird zunächst bei Raumtemperatur getrocknet und abschließend 48 h bei 250°C kalziniert.

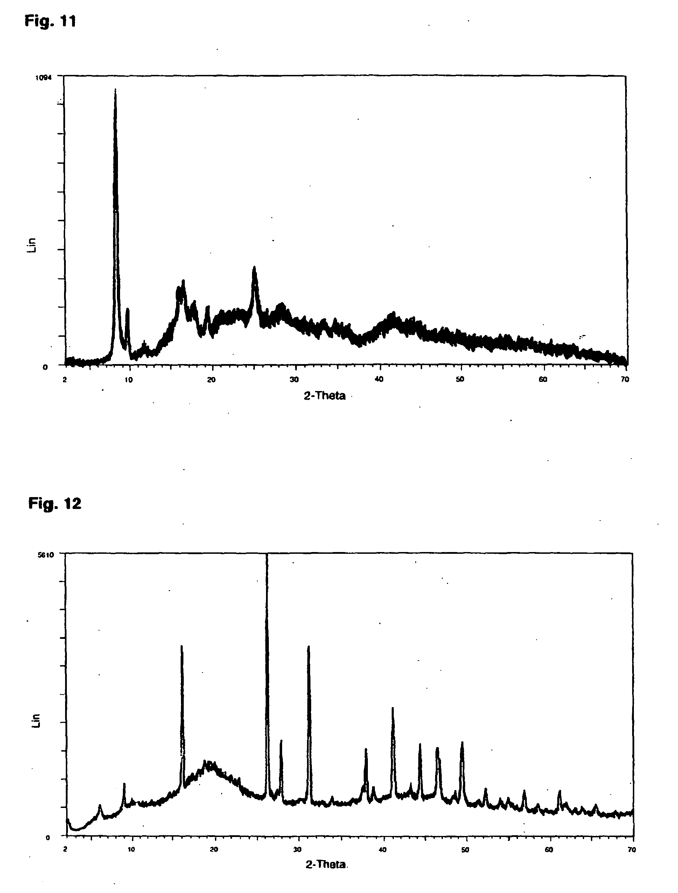

Es werden nach dem Trocknen 7,8 g bzw. nach dem Calcinieren 5,4 g Produkt erhalten. Die kalzinierte Form weist eine N₂-Oberfläche (nach Langmuir) von 294 m²/g auf. Fig. 10 zeigt das XRD der getrockneten, Fig. 11 das der kalzinierten Form.

### Vergleichsbeispiel 31 Herstellung eines Ca-5-tert-butyl-/sophthalsäure-MOFs

4,95 g 5-tert-butyl-Isophthalsäure und 9,75 g CaCl₂*6H₂O werden in 312,3 g DMF in einem Rührkolben suspendiert, auf 130°C erwärmt und unter Rühren 20 h bei diesen Bedingungen gehalten. Das ausgefallene Produkt wird abfiltriert, mit 1 x 50 ml DMF und 3 x 50 ml Methanol gewaschen und im Vakuumtrockenschrank 10 h bei 200°C getrocknet.

Es werden 4,9 g eines Produkt erhalten. Die N₂-Oberfläche beträgt 5 m²/g (nach Langmuir) - es handelt sich hierbei offensichtlich um einen MOF mit nur geringer Porosität oder sehr engen Poren. Das XRD ist in Fig. 12 gezeigt.

## Patentansprüche

1. Verfahren zur Herstellung eines porösen metallorganischen Gerüstmaterials den Schritte enthaltend
Umsetzung mindestens einer Metallverbindung mit mindestens einer mindestens zweizähnigen organischen Verbindung, die koordinativ an das Metall binden kann, in Gegenwart eines nicht-wässrigen organischen Lösemittels, wobei das Metall Be^{II}, Mg^{II}, Ca^{II}, Sr^{II}, Ba^{II}, Al^{III}, Ga^{III} oder In^{III} ist und wobei die organische Verbindung zumindest zwei Atome jeweils unabhängig ausgewählt aus der Gruppe bestehend aus Sauerstoff, Schwefel und Stickstoff aufweist, über die die organische Verbindung an das Metall koordinativ binden kann, wobei die Umsetzung unter Rühren und bei einem Druck von höchstens 2 bar (absolut) erfolgt und wobei nach der Umsetzung ein Calcinierungsschritt bei mehr als 250°C erfolgt.

2. Verfahren nach Anspruch 1, **dadurch gekennzeichnet, dass** die Umsetzung bei höchstens 1230 mbar (absolut), vorzugsweise bei Atmosphärendruck durchgeführt wird.

3. Verfahren nach Anspruch 1 oder 2, **dadurch gekennzeichnet, dass** die Umsetzung ohne zusätzliche Base erfolgt.

4. Verfahren nach einem der Ansprüche 1 bis 3, **dadurch gekennzeichnet, dass** die mindestens zweizähnige organische Verbindung eine Di-, Tri- oder Tetracarbonsäure ist.

5. Verfahren nach einem der Ansprüche 1 bis 4, **dadurch gekennzeichnet, dass** das Metall Al^{III} ist.

6. Verfahren nach einem der Ansprüche 1 bis 5, **dadurch gekennzeichnet, dass** das nicht-wässrige organische Lösemittel ein C₁₋₆-Alkanol, DMSO, DMF, DEF, Acetonitril, Toluol, Dioxan, Benzol, Chlorbenzol, MEK, Pyridin, THF, Essigsäureethylester, gegebenenfalls halogeniertes C₁₋₂₀₀-Alkan, Sulfolan, Glykol, NMP, gamma-Butyrolacton, alicyclische Alkohole, Ketone, Cycloketone, Sulfolen oder eine Mischung davon ist.

7. Verfahren nach einem der Ansprüche 1 bis 6, **dadurch gekennzeichnet, dass** nach der Umsetzung das gebildete Gerüstmaterial zusätzlich mit einem organischen Lösemittel nachbehandelt wird.

8. Verfahren nach einem der Ansprüche 1 bis 7, **dadurch gekennzeichnet, dass** die Metallverbindung nicht-ionisch und/oder das Gegenion zum Metallkation von einem protischen Lösemittel abgeleitet ist.

9. Poröses metallorganisches Gerüstmaterial erhältlich aus einem Verfahren nach einem der Ansprüche 1 bis 8.

10. Verwendung eines porösen metallorganischen Gerüstmaterials nach Anspruch 9 zur Aufnahme mindestens eines Stoffes zu dessen Speicherung, Abtrennung, kontrollierten Abgabe oder chemischen Umsetzung sowie als Trägermaterial.

11. Verwendung nach Anspruch 10, **dadurch gekennzeichnet, dass** das Metall Al^{III} oder Mg^{II} ist und das Gerüstmaterial zur Wasserstoffspeicherung dient.

## Claims

1. A process for preparing a porous metal-organic framework, which comprises the step
reaction of at least one metal compound with at least one at least bidentate organic compound which can coordinate to the metal, in the presence of a nonaqueous organic solvent, where the metal is Be^{II}, Mg^{II}, Ca^{II}, Sr^{II}, Ba^{II}, Al^{III}, Ga^{III} or In^{III} and the organic compound has at least two atoms which are selected independently from the group consisting of oxygen, sulfur and nitrogen and via which the organic compound can coordinate to the metal, with the reaction being carried out with stirring and at a pressure of not more than 2 bar (absolute) and a calcination step being carried out at more than 250°C after the reaction.

2. The process according to claim 1, wherein the reaction is carried out at not more than 1230 mbar (absolute), preferably at atmospheric pressure.

3. The process according to claim 1 or 2, wherein the reaction is carried out without additional base.

4. The process according to any of claims 1 to 3, wherein the at least bidentate organic compound is a dicarboxylic, tricarboxylic or tetracarboxylic acid.

5. The process according to any of claims 1 to 4, wherein the metal is Al^{III}.

6. The process according to any of claims 1 to 5, wherein the nonaqueous organic solvent is a C₁₋₆-alkanol, DMSO, DMF, DEF, acetonitrile, toluene, dioxane, benzene, chlorobenzene, MEK, pyridine, THF, ethyl acetate, optionally halogenated C₁₋₂₀₀-alkane, sulfolane, glycol, NMP, gamma-butyrolactone, alicyclic alcohols, ketones, cyclic ketones, sulfolene or a mixture thereof.

7. The process according to any of claims 1 to 6, wherein the framework formed is additionally after-treated with an organic solvent after the reaction.

8. The process according to any of claims 1 to 7, wherein the metal compound is nonionic and/or the counterion to the metal cation is derived from a protic solvent.

9. A porous metal-organic framework obtainable by a process according to any of claims 1 to 8.

10. The use of a porous metal-organic framework according to claim 9 for the uptake of at least one substance for the purposes of its storage, separation, controlled release or chemical reaction and also as support material.

11. The use according to claim 10, wherein the metal is Al^{III} or Mg^{II} and the framework is employed for the storage of hydrogen.

## Revendications

1. Procédé de fabrication d'un matériau de squelette métallo-organique poreux comprenant l'étape suivante :
la mise en réaction d'au moins un composé métallique avec au moins un composé organique au moins bidentate, qui peut se lier coordinativement au métal, en présence d'un solvant organique non aqueux, le métal étant Be^{II}, Mg^{II}, Ca^{II}, Sr^{II}, Ba^{II}, Al^{III}, Ga^{III} ou In^{III} et le composé organique comportant au moins deux atomes choisis chacun indépendamment dans le groupe constitué par l'oxygène, le soufre et l'azote, par lesquels le composé organique peut se lier coordinativement au métal, la réaction ayant lieu sous agitation et à une pression d'au plus 2 bar (absolu) et une étape de calcination ayant lieu à plus de 250 °C après la réaction.

2. Procédé selon la revendication 1, **caractérisé en ce que** la réaction est réalisée au plus à 1 230 mbar (absolu), de préférence à la pression atmosphérique.

3. Procédé selon la revendication 1 ou 2, **caractérisé en ce que** la réaction a lieu sans base supplémentaire.

4. Procédé selon l'une quelconque des revendications 1 à 3, **caractérisé en ce que** le composé organique au moins bidentate est un acide di-, tri- ou tétracarboxylique.

5. Procédé selon l'une quelconque des revendications 1 à 4, **caractérisé en ce que** le métal est Al^{III}.

6. Procédé selon l'une quelconque des revendications 1 à 5, **caractérisé en ce que** le solvant organique non aqueux est un alcanol en C₁₋₆, le DMSO, le DMF, le DEF, l'acétonitrile, le toluène, le dioxane, le benzène, le chlorobenzène, la MEK, la pyridine, le THF, l'ester éthylique de l'acide acétique, un alcane en C₁₋₂₀₀ éventuellement halogéné, le sulfolane, le glycol, la NMP, la gamma-butyrolactone, des alcools alicycliques, des cétones, des cyclocétones, le sulfolène ou un de leurs mélanges.

7. Procédé selon l'une quelconque des revendications 1 à 6, **caractérisé en ce que** le matériau de squelette formé est également post-traité avec un solvant organique après la réaction.

8. Procédé selon l'une quelconque des revendications 1 à 7, **caractérisé en ce que** le composé métallique est non ionique et/ou le contre-ion du cation métallique dérive d'un solvant protique.

9. Matériau de squelette métallo-organique poreux pouvant être obtenu par un procédé selon l'une quelconque des revendications 1 à 8.

10. Utilisation d'un matériau de squelette métallo-organique poreux selon la revendication 9 pour l'absorption d'au moins une substance pour son stockage, sa séparation, sa délivrance contrôlée ou sa réaction chimique, ainsi qu'en tant que matériau support.

11. Utilisation selon la revendication 10, **caractérisée en ce que** le métal est Al^{III} ou Mg^{II} et le matériau de squelette sert au stockage d'hydrogène.
